(19) Europäisches Patentamt
European Patent Office
Office européen des brevets

(11) Publication number: 0 302 303 A2

(12) **EUROPEAN PATENT APPLICATION**

(21) Application number: 88111666.9

(22) Date of filing: 20.07.88

(51) Int. Cl.4: C07D 471/04 , C07D 215/38 , A61K 31/47 , A61K 31/495 , //(C07D471/04,221:00,221:00), (C07D471/04,241:00,221:00)

(30) Priority: 22.07.87 US 76450
22.07.87 US 76469
06.04.88 US 178176

(43) Date of publication of application: 08.02.89 Bulletin 89/06

(84) Designated Contracting States: ES GR

(71) Applicant: SCHERING CORPORATION
2000 Galloping Hill Road
Kenilworth, New Jersey 07033(US)

(72) Inventor: Blythin, David J.
487 Mountain Avenue
North Caldwell New Jersey 07006(US)
Inventor: Shue, Ho-Jane
8 Hamilton Place
Pine Brook New Jersey 07058(US)
Inventor: Gala, Dinesh
9 Silvestor Court
East Brunswick New Jersey 08816(US)
Inventor: Steinman, Martin
3 Tuxedo Drive
Livingston New Jersey 07039(US)
Inventor: Ganguly, Ashit
96 Cooper Avenue
Upper Montclair New Jersey 07043(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Bicyclic compounds, their use as pharmaceuticals, their preparation, and intermediates useful in their preparation.**

(57) Bicyclic compounds are disclosed which are useful as anti-allergic, anti-inflammatory and/or cytoprotective agents and in the treatment of hyperproliferative skin disease. Pharmaceutical compositions and methods of treatment employing such compounds are also disclosed. Many of the compounds are novel; inventive methods for their preparation and for the preparation of intermediates, are also disclosed.

EP 0 302 303 A2

# BICYCLIC COMPOUNDS, THEIR USE AS PHARMACEUTICALS, THEIR PREPARATION, AND INTERMEDIATES USEFUL IN THEIR PREPARATION

The present invention relates to certain bicyclic compounds and to pharmaceutical compositions and methods of use employing such compounds, to their preparation, and to intermediates useful in their preparation.

An article by Bowman et al. entitled "The Synthesis of Some Dialkylamino-2-quinolones," Journal of the Chemical Society, pp. 1350-1353 (1964), discloses certain 1-alkyl-3-dialkylamino-4-hydroxy-2-quinolones. Mentioned in this article are 3-dimethylamino-4-hydroxy-1-phenyl-2-quinolone and 1-benzyl-3-dimethylamino-4-hydroxy-2-quinolone. No utility is mentioned in the article for such compounds.

Certain other 3-amino substituted quinolones are disclosed in Kappe et al., Monatshefte fur Chemie, 99, pp. 2157-2166 (1968); Merchant et al., Curr. Sci., 49(1), pp. 20-21 (1980); and Wittmann et al. Z. Naturforsch., B: Anorg. Chem., Org. Chem., 33B(12), pp. 1540-1546 (1978).

Processes for making certain bicyclic compounds and intermediates have been described in various publications, such as U.S. Patents 4,684,727, 4,628,055, 4,680,298, 4,492,702 and 4,452,800, Japanese Patent Disclosure 11,649, European Patent Application 0127135, and the article "Phosphorus Pentoxide in Organic Synthesis, III - A New Synthesis of Pyrido[2,3-d]pyrimidin-4(3H)-ones", O. Andersen and E. Pederson, Liebigs Ann. Chem., 1982, 1012-1015. It would be desirable to provide processes for preparing bicyclic compounds and their intermediates whose yields are as good as or better than methods previously taught. It would also be desirable to provide a process for preparing said bicyclic compounds and intermediates which requires even fewer steps than methods previously taught.

This invention concerns compounds of the formula I

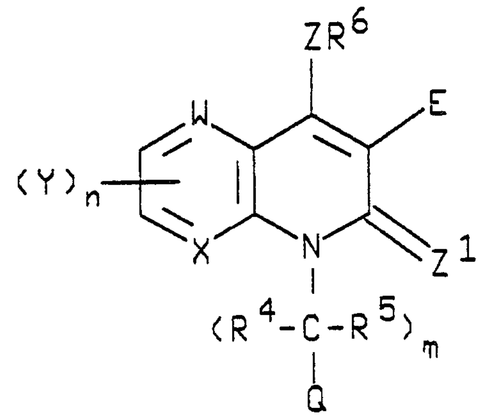

I

wherein:

W and X may be the same or different and each independently represents -CH= or -N=;

$Z^1$ and Z are the same or different and each independently represents 0 or S;

E is a group $-NR^1R^2$ or $-\overset{+}{N}R^1R^2R^3$;

$R^1$, $R^2$, $R^3$, $R^4$ and $R^5$ are the same or different and each may be independently selected from H, alkyl having from 1 to 12 carbon atoms, alkenyl having from 3 to 8 carbon atoms, alkynyl having from 3 to 8 carbon atoms, alkoxyalkyl having from 1 to 6 carbon atoms in the alkoxy portion and from 2 to 6 atoms in the alkyl portion thereof, hydroxyalkyl having from 2 to 8 carbon atoms, cycloalkyl having from 3 to 8 carbon atoms, acyloxyalkyl having from 1 to 6 carbon atoms in the acyloxy portion and from 2 to 8 carbon atoms in the alkyl portion thereof, and $-R^7-CO_2R^0$ wherein $R^7$ represents an alkylene group having from 1 to 6 carbon atoms and $R^0$ represents hydrogen or an alkyl group having from 1 to 6 carbon atoms, with the provisos that the OH of the hydroxyalkyl group and the acyloxy of the acyloxyalkyl group are not joined to the same carbon atom as another heteroatom and that, when $R^1$, $R^2$ and/or $R^3$ are alkenyl or alkynyl, there is at least one carbon-carbon single bond between the nitrogen atom and the carbon-carbon double or triple bond;

in addition, one of $R^2$ and $R^3$ can be an aryl group or an aromatic heterocyclic group, either of which can be substituted with one to three substituents Y as defined below;

in further addition, any two of $R^1$, $R^2$ and $R^3$, together with the adjacent nitrogen atom, can be joined together to represent a ring which can contain from 2 to 8 carbon atoms, said ring optionally containing an

-O-, -S- and/or $-NR^4-$ heteroatomic group (wherein $R^4$ is as defined above) and/or optionally containing a carbon-carbon double bond, said ring optionally being substituted with one to three additional substituents $R^8$ which may be the same or different and are each independently selected from OH with the proviso that OH is not on a carbon already joined to a hetero atom, -O-acyl having from 1 to 6 carbon atoms, hydroxyalkyl having from 1 to 8 carbon atoms, alkoxyalkyl having from 1 to 6 carbon atoms in the alkyl and alkoxy portions thereof, alkyl having from 1 to 6 carbon atoms, alkenyl having from 3 to 8 carbon atoms, alkynyl having from 3 to 8 carbon atoms, and $-COOR^9$ wherein $R^9$ represents H, alkyl or aryl, or any two $R^8$ substituent groups may combine to form a hydrocarbon ring having from 4 to 8 total carbon atoms;

in still further addition, all three of $R^1$, $R^2$ and $R^3$ can be joined together to represent a polycyclic hydrocarbon ring, which polycyclic ring can optionally be substituted by one to three substituent groups $R^8$ as defined above;

$R^6$ represents alkanoyl, $-CO-R^{10}$, $-CS-OR^{17}$, $-CS-NR^{15}R^{16}$, $-C(R^{11})_2-OR^{12}$, $-C(R^{11})_2-SR^{12}$ or $-C(R^{11})_2-NR^{12}R^{13}$ or, when E is $-\overset{+}{N}R^1R^2R^3$, $R^6$ represents $A^-$ alone or any of the foregoing groups together with $A^-$;

$R^{10}$ represents aryl, $R^{14}$, aromatic heterocyclic, $-OR^{14}$ or $-NR^{15}R^{16}$;

each $R^{11}$ represents H, alkyl, $-CCl_3$, $-COOR^9$ or aryl;

$R^{12}$ represents $-R^{14}$, $-CO-R^{13}$ or $-CS-R^{17}$;

$R^{13}$ represents H, alkyl or aryl;

$R^{14}$ represents alkyl of from 1 to 12 carbon atoms;

$R^{15}$ and $R^{16}$ each independently represents H, alkyl or aryl, or $R^{15}$ and $R^{16}$ together represent a divalent polymethylene group of from 4 to 6 carbon atoms, said polymethylene group being optionally substituted with a carboxy group or alkyl ester thereof;

$R^{17}$ represents $-R^{14}$ or aryl;

m is an integer of from 0 to 3;

n is an integer of from 0 to 2;

Q represents an aryl or an aromatic heterocyclic group which can optionally be substituted with 1 to 3 substituents Y as defined below;

each Y substituent is independently selected from hydroxy, alkyl having from 1 to 6 carbon atoms, halogen, $NO_2$, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, hydroxyalkyl having from 1 to 6 carbon atoms, $-S(O)_n-R^{18}$ (wherein $R^{18}$ represents alkyl having from 1 to 6 carbon atoms and n is as defined above), $-SO_2NH_2$, $-CO-R^{19}$ (wherein $R^{19}$ represents OH, $-NH-R^{18}$ or $-O-R^{18}$, where $R^{18}$ is as defined above), $-O-B-(O)_p-COR^{19}$ (wherein B represents an alkylene group having from 1 to 4 carbon atoms, p is 0 or 1, and $R^{19}$ is as defined above), $-NH_2$, -NHCHO, $-NH-CO-R^{19}$ (wherein $R^{19}$ is as defined above, with the proviso that it is not hydroxy), $-NH-COCF_3$, $-NH-SO_2R^{18}$ (wherein $R^{18}$ is as defined above), and $-NHSO_2CF_3$;

$A^-$ is one equivalent of a pharmaceutically acceptable anion or, when $R^6$ represents $A^-$ alone, then $A^-$ is an electron on Z; i.e., the group $ZR^6$ is $Z^-$ ;

together with their pharmaceutically acceptable salts with bases when $ZR^6$ is $Z^-$.

When $R^6$ represents $-C(R^{11})_2-OR^{12}$, the compounds are referred to herein as acetal ethers; when $R^6$ represents $-C(R^{11})_2-SR^{12}$, they are referred to herein as thioethers; and when $R^6$ represents $-C(R^{11})_2-NR^{12}R^{13}$ they are referred to herein as nitrogen-substituted derivatives. For further values of $R^6$ other than $A^-$ alone, the compounds are esters (e.g., alkyl esters, as when $R^6$ is $-O(CO)CH_3$ or $-O(CO)C_2H_5$). When $R^6$ represents $A^-$ alone, the compounds exist as zwitterions.

Compounds of formula I can be used for the preparation of pharmaceutical compositions useful in the erproliferative skin disease.

When used herein the terms listed hereinbelow, therwise indicated, are defined as follows:

halogen or halo - fluoro, chloro, bromo and iodo;

alkyl and alkoxy - comprise straight and branched carbon chains and, unless otherwise specified, contain from 1 to 6 carbon atoms;

alkenyloxy - comprises straight and branched carbon chains and, unless otherwise specified, contains from 3 to 8 carbon atoms and a carbon-to-carbon double bond;

alkynyloxy - comprises straight and branched carbon chains and, unless otherwise specified, contains from 3 to 8 carbon atoms and a carbon-to-carbon triple bond;

aryl - a carbocyclic group containing at least one benzene ring, with the aryl groups preferably containing from 6 to 15 carbon atoms, more preferably being phenyl or Y-substituted phenyl, e.g., phenyl, naphthyl, indenyl, indanyl, 4-chlorophenyl, 4-fluorophenyl, etc.;

aromatic heterocyclic - cyclic groups having at least one O, S and/or N hetero atom interrupting the ring structure and having a sufficient number of unsaturated carbon-to-carbon bonds, nitrogen-to-carbon bonds,

etc., to provide aromatic character, the aromatic heterocyclic groups preferably containing from 2 to 14 carbon atoms, e.g., pyridyl, furyl, thienyl, thiazolyl, imidazolyl, pyrimidinyl, pyrazinyl, pyridazinyl, 1,2,4-triazinyl, benzofuranyl, indolyl, pyrazolyl, oxazolyl, etc. Many such heterocyclic groups can be bonded via various positions on the ring and all such variations are included, e.g. 2- or 3-furanyl, 2-, 3- or 4-pyridyl, etc;

heteroatom - an atom other than carbon or hydrogen; in particular this term designates nitrogen and oxygen.

The compounds of the invention contain a $-(CR^4R^5)_m-$ substituent wherein each $R^4$ group and each $R^5$ group may vary independently. Thus, for example, when m equals 2 the following patterns of substitution (wherein hydrogen and $CH_3$ are used to represent any substituent, $R^4$ or $R^5$) are among those included: $-C(CH_3)_2CH_2-$, $-CH_2C(CH_3)_2-$, $-CH_2CH(CH_3)-$, $-CH(CH_3)CH_2-$, $-(C(CH_3)H)_2-$and the like. In addition when m equals 3, substituents such as $-C(CH_3)_2CH(C_2H_5)-CH_2-$, $-CH(CH_3)-CH_2CH(C_2H_5)-$, and $CH_2-CH(i-C_3H_7)CH-(C_2H_5)-$ are also included.

The $R^1$, $R^2$ and $R^3$ groups on the amino nitrogen in the compounds of the invention can be the same or different. In some compounds as noted above, $R^1$, $R^2$ and $R^3$ (or two of them) may together represent a heterocyclic ring system where the nitrogen of the amino group is a part of such ring, e.g., a monocyclic or bicyclic ring. Examples of suitable $-NR^2R^3$ groups include $NH_2$, the monosubstituted amino groups such as $-NH(CH_3)$, -NH(phenyl), $-NH(-CH_2-CH=CH_2)$, -NH(4-pyridyl), etc.; disubstituted amino groups such as $-N(CH_3)_2$, $-N(CH_2CO_2H)C(CH_2OH)_3$, etc.

Examples of suitable $-\overset{+}{N}R^1R^2R^3$ groups include a protonated amino group $-\overset{+}{N}H_3$ ; protonated monosubstituted amino groups such as $-\overset{+}{N}H_2(CH_3)$, $-\overset{+}{N}H_2(-CH_2-CH=CH_2)$, $-\overset{+}{N}H_2$(phenyl), $-\overset{+}{N}H_2$(4-pyridyl), etc.; protonated disubstituted amino groups such as $-\overset{+}{N}H(CH_3)_2$, $-\overset{+}{N}H(CH_2CO_2H)[C(CH_2OH)_3]$, etc.; quaternary amino groups such as $-\overset{+}{N}(CH_3)_3$, $-\overset{+}{N}(CH_3)_2$(phenyl) etc.; and protonated heterocyclic amino groups containing the nitrogen atom in the heterocyclic ring such as pyrrolidinium, 1-methylpyrrolidinium, piperidinium, 1-methyl-piperidinium,

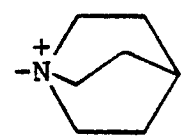 , etc.

As noted above, the compounds of the invention may include one to three Y substituents on the bicyclic ring system. Also, the Q group may include one or two Y substituents. Where there is more than one such Y substituent, they may be the same or different. Thus, compounds having combinations of different Y substituents are within the scope of the invention. Examples of suitable Y substituents include OH, methyl, chloro, bromo, methoxy, cyclohexyl, allyloxy, 2-propynyloxy, hydroxyethyl, methylthio, methylsulfonyl, carboxy, acetoxy, N-methylaminocarbonyl, acetoxymethoxy, acetamido, methylsulfonamido and the like.

Compounds of the present invention wherein $ZR^6$ is $Z^-$ and $R^1$ (or $R^2$ or $R^3$) is hydrogen can exist in a zwitterionic form, as illustrated below:

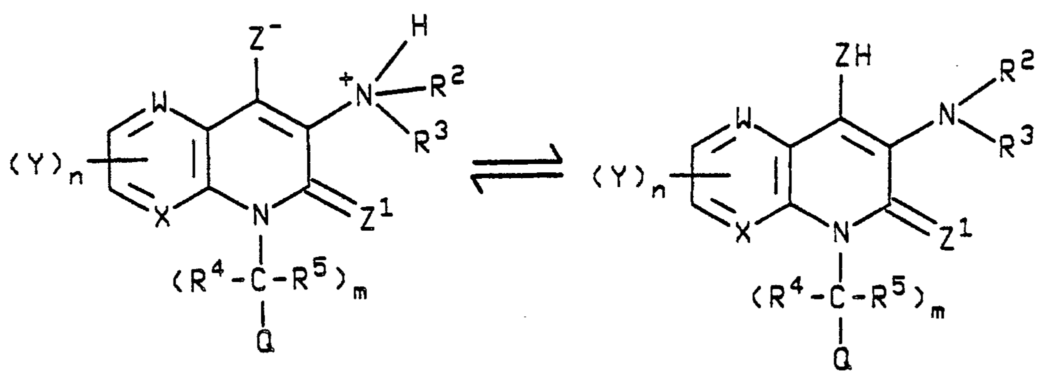

Exemplary compounds within the scope of the present invention are:

4-(4-methylbenzoyloxy)-1-phenyl-3-(1-pyrrolidinyl)-1,8-naphthyridin-2(1H)-one;

4-(N,N-diethylcarbamoyloxy)-1-phenyl-3-(1-pyrrolidinyl)-1,8-naphthyridin-2(1H)-one;

4-(N,N-dimethylcarbamoyloxy)-1-phenyl-3-(1-pyrrolidinyl)-1,8-naphthyridin-2(1H)-one;

and the pharmaceutically acceptable salts and solvates of said compounds.

The novel compounds of the present invention have the structural formula Ia or Ib

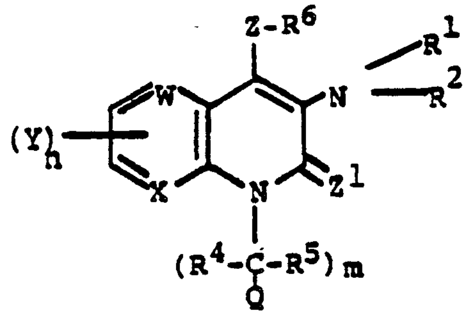

(Ia)

or

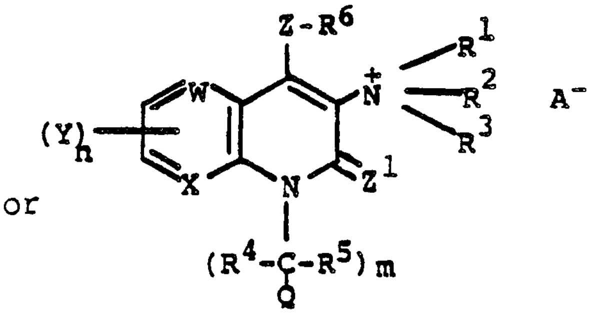

(Ib)

wherein the symbols W, X, Y, $Z^1$, Z, $R^1$, $R^2$, $R^3$, $R^4$, $R^5$, $R^6$, A, Q, n and m are as defined above, except that $R^6$ is not alkanoyl and does not represent or include $A^-$.

In a preferred subgenus of compounds at least one of W and X is N. More preferably, W is CH and X is N. Moreover, at least one of $Z^1$ and Z is preferably O, and m and n are preferably zero.

An additional preferred subgenus of compounds is represented by the structural formulae IIa and IIb

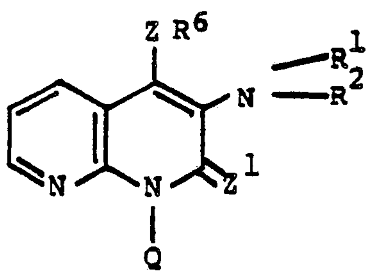

(IIa)

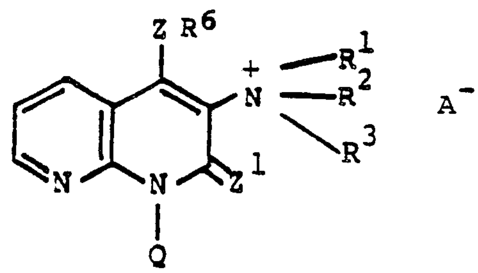

(IIb)

wherein $R^1$, $R^2$, $R^3$, $R^6$, Q, $Z^1$ and Z are as defined above (except that $R^6$ is not alkanoyl and neither represents nor includes $A^-$ ). Preferably, at least one of $Z^1$ and Z is 0. In addition, Q is preferably an aryl group such as a phenyl group, which may be optionally substituted with one to three Y groups, more preferably one or two Y groups.

Suitable Z-esters include aryl esters, aralkyl esters, aromatic heterocyclic esters such as furyl or pyridinyl esters, carbamates such as N,N-dialkyl-carbamates, proline carbamate esters, etc. Suitable ester groups exemplifying $ZR^6$ include -O(CO)phenyl, -O(CO)-p-tolyl, $-O(CO)N(C_2H_5)_2$, $-O(CO)C(CH_3)_3$,

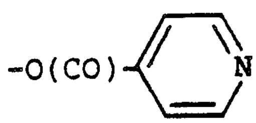 , 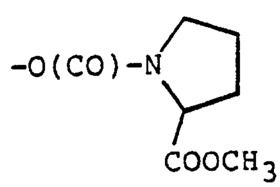 , etc.

Examples of suitable Z-acetal ethers include those wherein both $R^{11}$ groups are H and where $R^{12}$ is $C_{8-12}$ alkyl such as n-octyl or $R^{12}$ is CO-alkyl such as $(CO)C(CH_3)_3$. In examples of suitable thioethers and nitrogen-containing compounds, $ZR^6$ includes $-CH_2-S-$alkyl wherein the alkyl group contains 8-12 carbon atoms, e.g., n-octyl, and $-CH_2-$NHalkyl wherein the alkyl group contains from 1-12 carbon atoms, e.g., n-octyl.

The invention also concerns (1) pharmaceutical compositions comprising a compound of formula Ia or

Ib as defined above in combination with a pharmaceutically acceptable carrier and (2) methods of using the compounds of formula I, especially Ia or Ib, as defined above to treat hyperproliferative skin disease, or of using the compounds of formula Ia or Ib to treat asthma, allergic reactions, inflammation or peptic ulcers by administering such a compound to a mammal in an amount effective for such purpose.

The compounds of formula I wherein E is $-\overset{+}{N}R^1R^2R^3$ and $ZR^6$ is $Z^-$ are zwitterionic or inner salts, i.e., they are both positively and negatively charged. They form pharmaceutically acceptable salts, i.e., pharmaceutically acceptable acid addition or basic salts, which are also part of the present invention. Examples of suitable acid addition salts include the chloride (from hydrochloric acid), methyl sulfate, (from methyl sulfuric acid) sulfate (from sulfuric acid) and bromide. Basic salts can be formed when at least one of $R^1$, $R^2$ and $R^3$ is H. Examples of suitable basic salts include sodium, potassium or calcium salts (from their corresponding hydroxides).

The compounds of the invention of formula I can exist in unsolvated as well as solvated forms, including hydrated forms, e.g., a hemihydrate. In general, the solvated forms, with pharmaceutically acceptable solvents such as water, ethanol and the like are equivalent to the unsolvated forms for purposes of the invention.

Certain compounds of the invention may exist in isomeric and tautomeric forms. The invention includes all such isomers and tautomers - the isomers both in pure form and in admixture, including racemic mixtures.

Those compounds of the invention that are zwitterionic provide good solubility in physiological fluids, such as blood, plasma, saliva, etc., and in general in polar solvents, such as water and ethanol, which can be used in compositions for delivering the compounds to patients. This characteristic is advantageous in that the compounds are expected to be more easily absorbed gastrointestinally and therefore provide good activity when administered orally.

The invention includes various pharmaceutical methods, in particular a method for treating asthma/allergic reactions in a mammal, a method for treating inflammation in a mammal, and a method for treating peptic ulcers in a mammal, which comprise administering a compound of the invention or a pharmaceutical composition containing it to the mammal, in an amount sufficient to provide respectively an anti-asthma/anti-allergic effect, an anti-inflammatory effect, or a cytoprotective effect.

Compounds of the present invention wherein $ZR^6$ represents $Z^-$ may be prepared from a compound of formula (III):

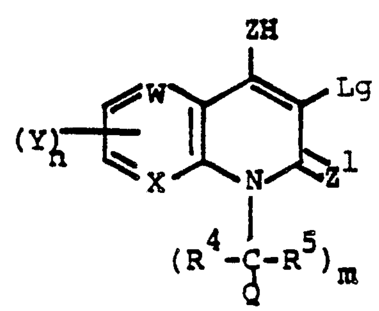

(III)

wherein $R^4$, $R^5$, Q, X, Y, W, $Z^1$, Z, m and n are as defined above and Lg is a substituent known to those skilled in the art as a "leaving group", by treatment with an amine of the formula

$NR^1R^2R^3$ (IV)

(wherein $R^1$, $R^2$, and $R^3$ are as defined above), preferably with heating in a suitable solvent, such as pyridine, dimethylformamide, hexamethylphosphoramide, 2,6-lutidine, dimethylacetamide and the like. The reaction, depending upon the reactants chosen, can be performed at temperatures of about 60°C up to the reflux temperature of the particular solvent.

For purposes of the invention, a "leaving group" is defined as a substituent which may be readily displaced, usually carrying a negative charge. Representative examples of suitable leaving groups include chloride, bromide, iodide, trifluoroacetoxy, methanesulfonyloxy, trifluoro-methanesulfonyloxy, p-toluenesulfonyloxy, $-\overset{+}{I}$-Ar, and the like. A preferred leaving group is bromide.

The compound of formula IV above is generally a secondary or tertiary amine, i.e., one in which at most one of the groups $R^1$, $R^2$ and $R^3$ is hydrogen. Such materials are readily obtainable either commercially or

by methods well known to one of ordinary skill in the art.

The intermediates of formula III above either are known or can be prepared from corresponding 3-unsubstituted derivatives which are disclosed, for example, in U.S. patent No. 4,492,702, the disclosure of which is incorporated herein by reference. For example, a compound of the formula (V)

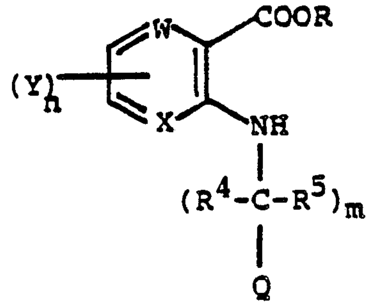

(V)

(wherein Q, $R^4$, $R^5$, Y, W, X, n and m are as defined herein and R is any convenient alkyl group) may be reacted with a compound of structural formula VI

$CH_3CO_2R$ (VI)

(wherein R is again, for example, an alkyl group) to produce a compound of the formula VII directly

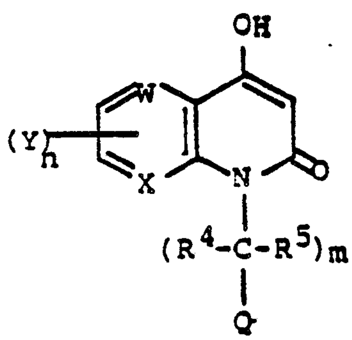

VII

This reaction is preferably accomplished by contacting the two reactants V and VI in the presence of a base such as a metal alkoxide, e.g., potassium tertiary butoxide or the like, at an elevated temperature, e.g., 60° to about 160°C, for a sufficient time until the reaction is substantially completed. The reaction is preferably conducted in an inert atmosphere such as nitrogen. Alternatively, the reaction may be conducted in the presence of a non-reactive solvent such as toluene, xylene, etc.

The compounds of formula VII above can be reacted with a suitable agent to provide the leaving group in the 3-position on the ring. For example, direct bromination of the compound of formula VII above will provide a compound of formula III above where Lg equals Br. As another example, reaction of the compound of VII above with iodosobenzene results in the formation of a compound of formula III where Lg is $-\overset{+}{I}$-Ph.

Esters, acetal ethers, thioethers and nitrogen-substituted derivatives of the present invention (according to the meanings of $R^6$) may be prepared by reacting a compound of structural formula VIII with a compound of formula IX:

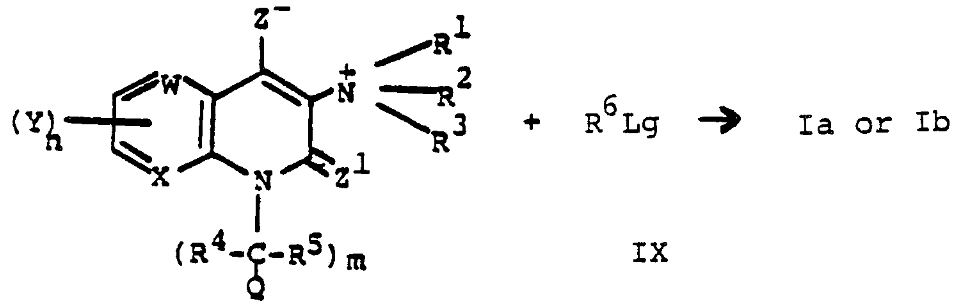


IX

VIII

wherein $R^1$, $R^2$, $R^3$, $R^4$, $R^5$, $R^6$, Q, X, Y, W, $Z^1$, Z, m and n are as defined above (except that $R^6$ neither represents nor includes $A^-$ ) and Lg is an appropriate leaving group such as halo, e.g., chloro. For example, suitable reagents include an appropriate anhydride or acid halide to prepare the esters, or a compound $LgC(R^{11})_2OR^{12}$, $LgC(R^{11})_2SR^{12}$ or $LgC(R^{11})_2\text{-}NR^{12}R^{13}$ to prepare the acetal ethers, thioethers or nitrogen-substituted compounds. Exemplary reagents include acetyl chloride, pivaloyl chloride, N,N-diethyl carbamoyl chloride, methoxymethyl chloride, pivaloyloxymethyl chloride, N-(chloromethyl)-benzamide, chloromethyl phenyl sulfide, etc. Preferably, the reaction is performed in a basic solvent such as pyridine or 2,6-lutidine (with or without the addition of dimethylaminopyridine (DMAP)) or in a neutral solvent in the presence of an organic base such as triethylamine. Sometimes, it is desirable to add a halide-exchange reagent such as NaI or KBr to the reaction mixture to produce a more reactive leaving group.

The invention further provides an inventive process -- or a group of cognate inventive processes -- for the preparation of the compounds of formula I hereinbefore defined, and for the preparation of intermediates.

One such inventive process is directed toward the preparation of a bicyclic compound of the formula

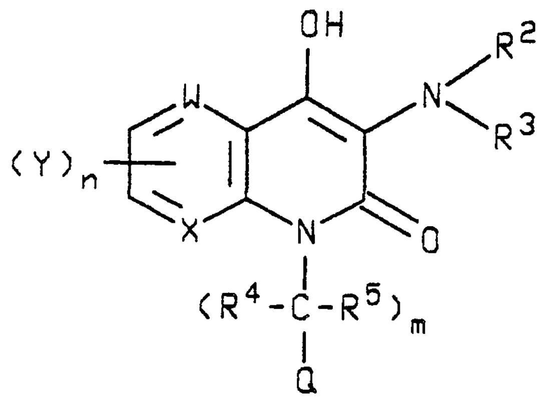

(X)

wherein W, X, $R^2$, $R^3$, $R^4$, $R^5$, m, n, Q and Y are as defined above;
it comprises the step of cyclising, in the presence of a base and a solvent, a compound of the formula

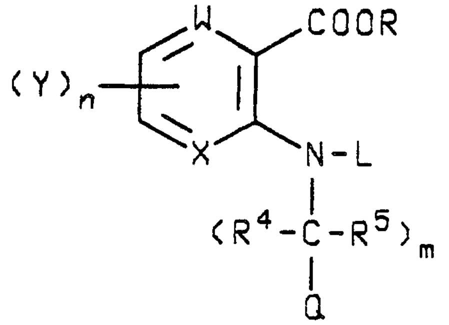

(VA)

wherein L is H or a group of the formula
$-CO-CH_2-NR^2R^3$ (VB),
$R^2$, $R^3$, $R^4$, $R^5$, Q, W, X, Y, m and n are as defined above,
and R is any of the values for $R^4$ or $R^5$ with the proviso that R is not hydrogen,
intramolecularly when L represents the group of the formula (VB),
and intermolecularly with an amino-substituted acetic acid derivative of the formula
$ROOC-CH_2-NR^2R^3$ (XI)
wherein $R^2$ and $R^3$ are as defined hereinbefore and R is as defined above, when L represents H.

This process provides the compound of formula (VII) in high yield and good purity with little formation of by-products. Reaction conditions are mild, especially for the intramolecular cyclization. Furthermore, the compound of formula (X) is readily recovered from the reaction mixture.

When L represents the group of formula (VB), this process comprises the step of contacting an amino acetamide compound of the formula

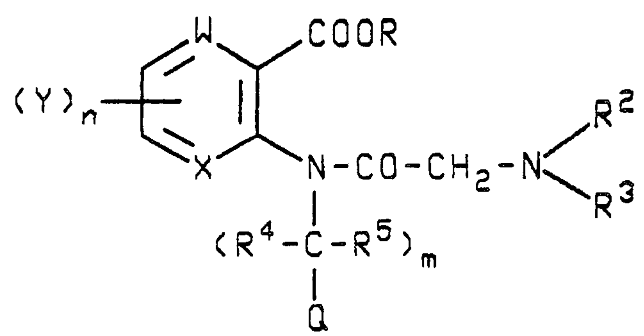

(VC) ,

wherein Y, W, X, $R^2$, $R^3$, $R^4$, $R^5$, Q, m and n are as defined hereinbefore, and
R is any of the values for $R^4$ and $R^5$ with the proviso that R is not hydrogen,
with a base effective to remove a proton selectively from the methylene group (i.e. $-CH_2-$) of said amino acetamide compound (VC) in order to cyclize said compound intramolecularly to the bicyclic compound of formula (X). This embodiment is referred to as Process A below.

In the amino acetamide compound of formula (VC) and the product of formula (X), preferably Y is hydrogen, X is nitrogen and W is CH, R is alkyl, more preferably methyl, m is 0, $R^2$ and $R^3$ are joined together to represent a ring containing four carbon atoms, and Q is phenyl. Preferably the base is potassium t-butoxide.

When L represents H, this process comprises the step of contacting a secondary substituted amine of the formula

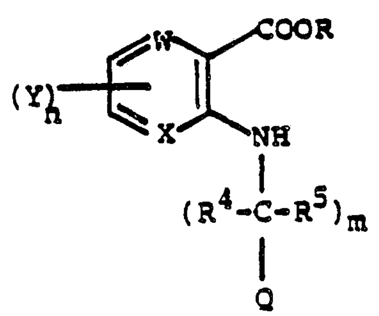

(V)

wherein Y, X, W, $R^4$, $R^5$, Q, m and n are as defined hereinbefore;
with an amino-substituted acetic acid derivative of the formula:
$ROOC-CH_2-NR^2R^3$ (XI)
wherein $R^2$ and $R^3$ are as defined hereinbefore, and R is as defined above;
with a base effective to cyclize said secondary substituted amine (V) with said amino-substituted acetic acid derivative (XI) to give the desired bicyclic compound (X). This embodiment is referred to as Process B below.

In the substituted secondary amine (V), preferably Y is hydrogen, X is nitrogen, W is CH, R is alkyl,

preferably methyl, Q is phenyl and m is zero. In the amino-substituted acetic acid derivative (XI), preferably R is alkyl, especially ethyl, and $R^2$, $R^3$ and the adjacent N atom are joined together to represent a ring containing four carbon atoms.

A preferred base is sodium hydride or potassium t-butoxide.

It is possible that this process proceeds through an intermediate of one of the following formulae

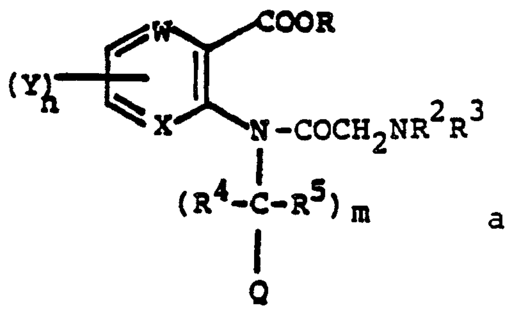

and

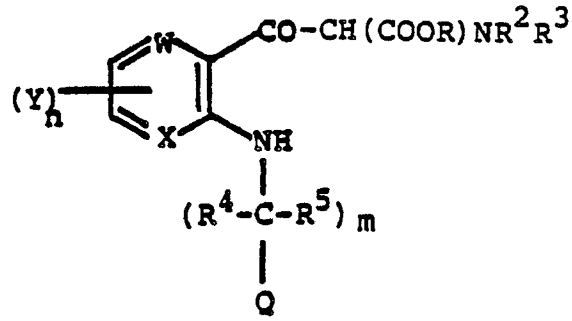

(wherein the various symbols have the meanings given above) and, indeed, that a compound of one of these formulae could even be isolated as an intermediate and then cyclized in a separate step. However, this process is not restricted by theoretical considerations; any process comprising the step of condensing compounds of the formulae (V) and (XI) to provide a compound of the formula (X) falls within this feature of the invention.

The intermediate of the formula (VC) can be prepared by a process wherein a secondary amine compound of the formula

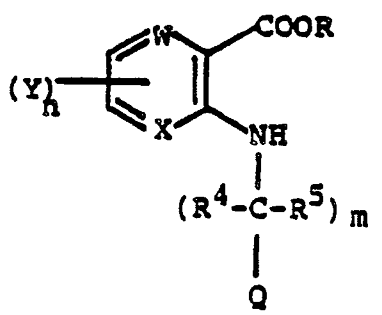

(V)

wherein Y, W, X, R, $R^4$, $R^5$, Q, m and n are as defined hereinbefore;

is contacted with a substituted acetic acid derivative of the formula

Hal-$CH_2$COLg

wherein Hal is a halogen, and

Lg is a leaving group, e.g. halogen, sulfonyloxy, especially tosyloxy or mesyloxy, or acetoxy, haloacetoxy or 2,2-dimethylpropionyloxy;

in the presence of a proton-accepting compound;

followed by reaction of the intermediate of the formula (XII)

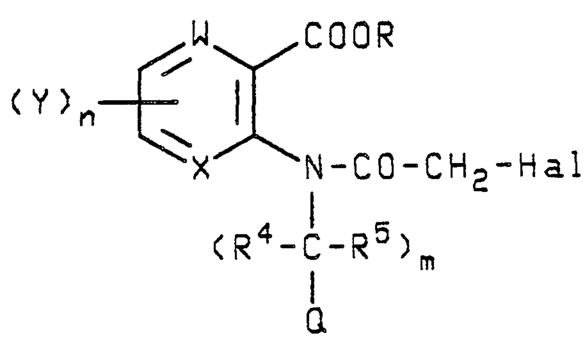

(XII)

with an amine $HNR^2R^3$, wherein $R^2$ and $R^3$ are as defined above.

The preparation of the intermediate of the formula (XII) in this process and of the starting material of the formula (VC) constitutes a further embodiment of the invention. This preparation of the intermediate of formula (XII) is referred to as Process C below.

In the secondary amine compound of the formula (V), preferably Y is hydrogen, X is nitrogen, W is CH, R is alkyl, more preferably methyl, m is zero, and Q is phenyl.

In the substituted acetic acid derivative of formula Hal-$CH_2$-COLg, preferably Lg is halogen, more preferably chloro, or haloacetoxy, especially chloroacetoxy. The proton-accepting compound is preferably an epoxide, most preferably propylene oxide. This process uses relatively mild reaction conditions to provide the compounds of formulae (XII) and (VC) in high yield and good purity, with little formation of by-products.

The inventive processes of the present invention can be schematically illustrated as follows:

Process B

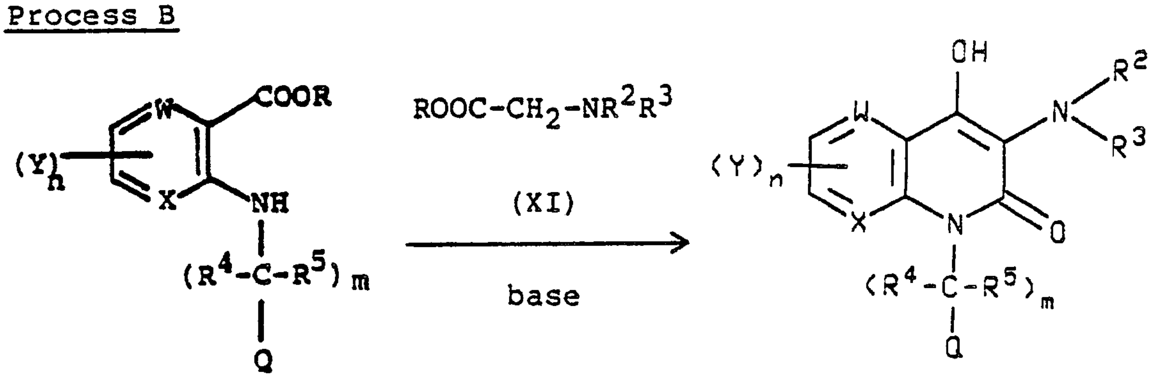


(V)

(X)

Process A

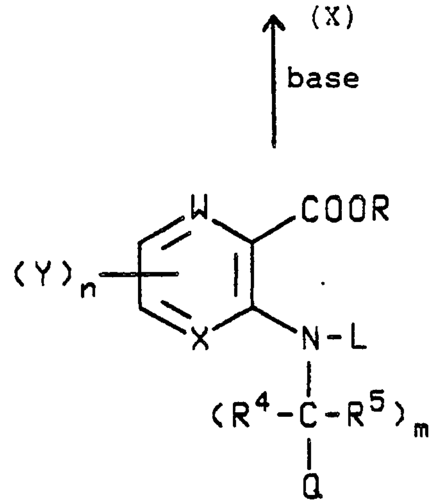


(VA) , L=(VB)

Process C

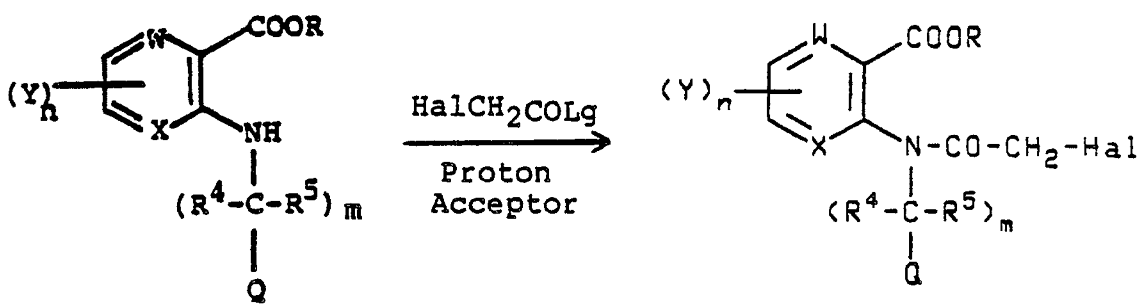


(V)

(XII)

In process A, the amino acetamide of formula (VA) (wherein L is (VB)) can be contacted with the base at temperatures ranging from about -100° C to about 100° C, preferably from about -70° to about 40° C, for about 5 minutes to about 24 hours or more, depending upon the base employed. The reactants are preferably stirred at ambient pressure in the presence of a solvent, e.g., a chlorinated hydrocarbon such as carbon tetrachloride ($CCl_4$), methylene chloride ($CH_2Cl_2$), or dichloroethane; aliphatics such as C-4 to C-20

alkanes, cyclic or acyclic; aromatics such as benzene, toluene, xylene, alkylbenzenes and the like; ethers such as diethyl ether and tetrahydrofuran (THF) and t-butyl-methyl ether; dimethylformamide (DMF) or dimethylsulfoxide (DMSO); or mixtures thereof.

The base used in Process A is any substance which will remove a proton from the methylene ($-CH_2-$) group of the moiety

$-\overset{|}{N}-CO-CH_2-NR^2R^3$,

and thereby intramolecularly cyclize the amino acetamide compound of formula (VA).

Bases which can be used in process A are generally non-aqueous bases of the organo-alkali metal type, such as primary, secondary and tertiary butyl lithium, lithium diisopropyl amide, lithium hexamethyldisilazane, sodium hexamethyldisilazane, potassium hexamethyldisilazane, potassium t-butoxide or sodium methoxide; inorganic bases of alkali and alkaline earth metals including carbonates such as sodium, potassium and cesium carbonates, or hydroxides such as sodium and potassium hydroxides; hydrides such as sodium or potassium hydride can also be used. Preferably the base is sodium hydride or sodium methoxide, and preferably potassium t-butoxide. The amount of base is used in mole ratios ranging from about 20:1 to 2:1, (moles of base:mole amino acetamide (VC)).

After the reaction is completed, the desired bicyclic compound of formula (X) is recovered by conventional separatory and recovery methods such as chromatography, distillation, crystallization and the like.

The reactants in process B can be contacted neat, although preferably a solvent is used.

The bases and solvents used in process B are essentially the same as those described above in process A. The secondary substituted amine of formula (V) is stirred with the amino-substituted acetic acid derivative of formula (XI) at temperatures ranging from about -40 to about 200 °C, preferably from about 25 to about 180 °C, until the reaction is substantially completed, preferably from about 1 hour to about 48 hours.

The base is employed in mole ratios ranging preferably from about 15:1 to 3:1 (moles base:mole secondary substituted amine (V)).

After the reaction is completed, the desired bicyclic compound of formula (X) is recovered by conventional separatory and recovery methods as indicated above for process A.

In process C, the substituted acetamide compound of formula (XII) is prepared by contacting a secondary amine compound of formula (V) with a substituted acetic acid derivative of formula $HalCH_2COLg$ (where Hal and Lg are as defined above) in the presence of a proton acceptor in amounts and under conditions effective to give the substituted acetamide compound (III).

The substituted acetic acid derivative $HalCH_2COLg$ (where Hal and Lg are as defined above) is preferably used in excess.

The term "proton acceptor" is defined as a compound which accepts either a proton from an acid or free protons in the reaction mixture, but generally will not accept a proton from the methylene group of the group of the formula

$Q-(CR^4R^5)_m-\overset{|}{N}-CO-CH_2-X$;

or of the compound of the formula

$Hal-CH_2-COLg$

wherein $-CH_2-$ is the methylene group. The proton acceptor should be compatible with the reactants and can be an organic base, especially a primary amine such as methylamine, n-butylamine, sec-butylamine, t-butylamine, benzylamine, cyclohexylamine, ethylamine, or ethylenediamine; a secondary amine or a compound containing at least one secondary amine group such as dimethylamine, N-methylpropylamine, diethylamine, or diisopropyl amine; a tertiary amine such as trimethylamine, N,N-dimethyl-n-propylamine, N-methylpiperidine, N,N-diethylbutylamine, or triethylamine; a heterocyclic base such as piperazine, piperidine, pyrrolidine, morpholine, or pyridine, or an inorganic base such as ammonia and those bases of alkali or alkaline earth metals discussed hereinbefore. The proton acceptor can also be an epoxide, especially one of the formula:

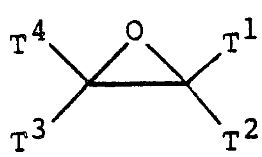

wherein $T^1$, $T^2$, $T^3$ and $T^4$ independently represent hydrogen atoms or alkyl groups. Representative epoxides suitable as proton acceptors include but are not limited to ethylene oxide and especially propylene oxide. Alternatively, the epoxide can be prepared in situ in the reaction mixture. The proton acceptor can also include mixtures of the base and epoxide.

The proton acceptor is used in amounts effective to scavenge the liberated protons, i.e. in a few moles excess, e.g. about 20:1 to 1:1 mole (moles proton acceptor:mole secondary amine of formula (V)).

Process C can be conducted neat, although a solvent is preferred. The reaction is carried out until it is substantially complete, e.g. from about 15 minutes to about 4 hours. Generally the reactants are stirred during the process.

Optionally, the process can be conducted in the presence of a catalyst such as N,N-dimethylaniline, 4-dimethylaminopyridine or phase-transfer catalysts. Phase transfer catalysts suitable for carrying out the process of the present invention include quaternary ammonium and phosphonium salts, ethers, and tertiary amines, such as tributyl amine or those described in U.S. Patent 3,969,360.

The catalyst can be used in an amount ranging from about 0.0001 to about 0.5 parts by weight of reactants, preferably from about 0.001 to about 0.1 parts by weight.

Process C is generally carried out at about -40° to about 200°C, preferably from about 0 to about 80°C, depending upon the boiling point of the epoxide, solvent or starting materials, and preferably at ambient pressure.

The compounds having structural formula I above wherein $Z^1$ and Z are oxygen may be converted into the corresponding compounds wherein $Z^1$ and/or Z are sulfur by known methods. For example, treatment with Lawesson's Reagent [2,4-bis(4-methoxyphenyl)-1,3-dithia-2,4-diphosphetane-2,4-disulfide] in an appropriate inert organic solvent such as hot toluene will effect this conversion. The isomeric and tautomeric forms can be purified by chromatography of the reaction mixture.

When Z should be sulfur, this conversion should be carried out on a product wherein $ZR^6$ is $O^-$ or on an intermediate lacking the group $R^6$.

The compounds of formulae Ia and Ib may be employed as anti-allergy agents in the treatment of, for example, asthma, allergic or seasonal rhinitis, and/or chronic bronchitis.

The anti-allergic properties of these compounds are shown by tests which measure a compound's inhibition of anaphylactic bronchospasm in sensitized guinea pigs having antigen induced bronchoconstriction.

In one such test procedure, male Hartley guinea pigs (250-300 g) are sensitized with 5 mg ovalbumin injected i.p. and 5 mg injected s.c. in 1 ml saline on day 1 and 5 mg ovalbumin injected i.p. on day 4. The sensitized animals are used 3-4 weeks later at which time they weigh 450-500 g.

The sensitized guinea pigs are fasted overnight and the following morning are anesthetized with 0.9 ml/kg i.p. of dialurethane (0.1 g/ml diallylbarbituric acid, 0.4 g/ml ethylurea and 0.4 g/ml urethane). The trachea are cannulated and the animals are ventilated by a Harvard rodent respirator at 50 strokes/minute with a stroke volume of 5 ml. A side arm to the tracheal cannula is connected to a Harvard pressure transducer to obtain a continuous measure of intratracheal pressure which is recorded on a Harvard polygraph. The jugular vein is cannulated for the i.v. administration of substances. The animals are challenged with antigen (0.5% ovalbumin) as an aerosol generated from a DeVilbiss Model 65 ultrasonic nebulizer and delivered through the tracheal cannula for 30 seconds. Bronchoconstriction is measured as the peak increase in intratracheal pressure occurring within 5 minutes after antigen challenge.

The sensitized guinea pigs are injected i.v. with 1 mg/kg propranolol, 5 mg/kg indomethacin and 2 mg/kg mepyramine given together in a volume of 1 ml/kg. Fifteen minutes later the animals are challenged with nebulized ovalbumin. Test compounds are administered orally 2 or 8 hours before challenge with ovalbumin. Suppression of anaphylactic bronchospasm is expressed as a percent inhibition of the peak increase in intratracheal pressure by comparison to a vehicle-treated control group. Results for representative compounds of the invention are shown in Table I below:

Table I

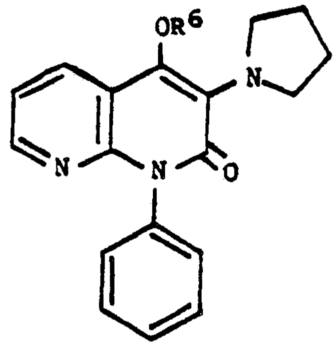

| $R^6$ | Dose (mg/kg p.o.) | % Inhibition After 2 hr | 8 hr. |
|---|---|---|---|
| 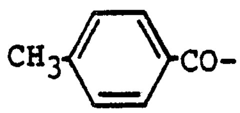 | 5 | 30 | 0 |
| $(C_2H_5)_2N-CO-$ | 5 | 48 | 32 |

The results demonstrate that the compounds of the formula Ia are effective inhibitors of allergic reactions, and that some provide a relatively long duration of action.

The compounds are effective non-adrenergic, non-anticholinergic, antianaphylactic agents. When administered orally they are active at doses from about 0.1 to 10 mg/kg of body weight; when administered parenterally, e.g., intravenously, the compounds are active at dosages of from about 0.05 to 5 mg/kg body weight; when administered by inhalation (aerosol or nebulizer) the compounds are active at dosages of about 0.25 to 5 mg per puff, and one to four puffs may be taken every 4 hours.

The compounds of this invention are also useful for the treatment of inflammation. Thus, they are useful in the treatment of arthritis, bursitis, tendonitis, gout and other inflammatory conditions. The anti-inflammatory use of the compounds of the present invention may be demonstrated by the Reversed Passive Arthus Reaction (RPAR) Synovitis technique as set forth below using male Lewis rats (obtained from Charles River Breeding Laboratories) weighing 200-250 grams and the RPAR Paw technique as also described below. The potency of the compounds is determined using indomethacin as the standard. On the basis of the test results, an oral dosage range of about 5 milligrams per kilogram of body weight per day to about 50 milligrams per kilogram of body weight per day in divided doses taken at about 4 hour intervals is recommended.

The dosage to be administered and the route of administration depend upon the particular compound used, the age and general health of the patient and the severity of the inflammatory condition. Thus, the dose ultimately decided upon must be left to the judgment of a trained health-care practitioner.

## RPAR Synovitis Technique

A Lewis rat is dosed orally with drug or placebo one hour prior to intravenous administration of 2.28 mg of bovine serum albumin (BSA) in 0.2 cc of pyrogen-free saline followed by the intraarticular injection of 0.54 mg of rabbit anti-BSA antibody in 0.03 cc of pyrogen-free saline into one knee joint. The contralateral knee is injected with 0.03 cc of pyrogen free saline. All injections are made with the animal under light ether anesthesia. Three hours later the rat is again dosed orally with drug or placebo. All drug doses are split. That is, one-half of the dose is administered before lesion induction and one-half is administered after lesion induction.

The following morning (about 17 hours after lesion induction) the rat is killed and both knee joints are

exposed. The subpatellar areolar tissue with attendant synovium is excised and weighed. Differences between the weight of antibody and saline injected knees are considered to represent the inflammatory response for each animal (delta synovial weight). Differences in delta synovial weight between lesion controls and drug-treated rats are evaluated for statistical significance with an analysis of variance. Relative potencies are determined with a linear regression analysis.

Reversed Passive Arthus Response (RPAR) PAW

Animals, Materials and Methods

Male Lewis inbred albino rats weighing 180-200 grams obtained from Charles River Breeding Laboratories are used in these experiments. The rats are housed 3 animals/cage and food and water are allowed ad libitum. The animals are numbered 1-3 in each cage and color marked for identification purposes.

Drug and Reagent Preparation

All reagents and drugs are prepared just prior to the study. Crystallized and lyophilized bovine serum albumin (BSA), available from Sigma Chemical Company, is solubilized without shaking in cold, sterile, pyrogen-free saline (10 mg/ml). Lyophilized anti-bovine serum albumin (IgG fraction), obtained from Cappel Laboratories, is suspended in sterile distilled water and diluted with cold, pyrogen-free saline (PFS) just prior to use. The final concentration of anti-bovine serum albumin is 0.5 mg/ml of PFS. Both BSA and anti-BSA solutions are iced during use. Drugs are suspended or solubilized in an aqueous solution of methyl cellulose (MC) with an homogenizer just prior to administration.

Drug Administration and Induction of Inflammation

Groups of animals (6/group) are dosed with drug in MC by gavage once daily for 3 days. The last dose is administered one hour prior to sensitization with BSA. Controls are given MC alone and a drug-standard is usually included in each assay for verification purposes. Drugs are prepared and diluted so as to provide a dose for a 200 gram animal which is equivalent to the mg/kg dose for each experiment. Thus each rat receives an oral dose in a volume of approximately 2.0 cc. One hour after the last dose the animals are lightly anesthetized with ether and "sensitized" by injection of 0.2 ml of PFS containing 1.0 mg of BSA into the penile vein. One hour later, the animals are "challenged" in the right rear paw with subplantar injections of 0.2 ml of PFS containing 0.1 mg of anti-BSA. Immediately after the subplantar injection, the right paw is dipped (up to the lateral maleolus) into the mercury well of a plethysmograph. The volume of mercury displaced is converted to weight and recorded. This value is considered to be the control reading for the animal. Paw volumes are subsequently recorded with a plethysmograph during the development of the inflammation at 2 and 4 hours post-challenge.

Results

Results are expressed by the change in paw volume ($\Delta$ paw volume) from the control reading for each animal to that recorded 2 and 4 hours post-challenge. All drug treated groups are compared to the MC control for significant differences with an analysis of variance. Differences from control in drug-treated groups are expressed as percent change from control. For example, 4-(N,N-diethylcarbamoyl)-1-phenyl-3-(1-pyrrolidinyl)-1,8-naphthyridin-2(1H)-one given at an oral dose of 25 mg/kg inhibited the paw edema by 54% and 17% at 2 hours and at 4 hours, respectively.

The compounds of this invention are also useful in the treatment of peptic ulcers. They display chemotherapeutic activity which enables them to relieve the symptoms of peptic ulcer disease, stress ulceration, and promote healing of gastric and/or duodenal ulcers. The antiulcer activity of the compounds of this invention is identified by tests which measure the cytoprotective effect in rats. The compounds are also useful as conjunctive therapeutic agents for coadministration with such antiinflammatory/analgesic agents as aspirin, indomethacin, phenylbutazone, ibuprofen, naproxen, tolmetin and other agents. The compounds of this invention prevent the untoward side effects of irritation and damage to the gastrointestinal tract caused by such agents.

The compounds of this invention are evaluated for their antiulcer activity characteristics by standard biological testing procedures such as the indomethacin-induced ulcer and/or ethanol-induced ulcer assays detailed below:

Indomethacin-Induced Ulcer Assay

Male Charles River CD rats (250-260g) are fasted overnight. The test compound is administered orally in methyl cellulose vehicle (2 ml/kg) to the animals one hour prior to indomethacin 10 mg/kg p.o. The rats are sacrificed by $CO_2$ asphyxiation 4 hours after administration of indomethacin. The stomachs are examined under a magnifying glass for lesions (Chiu et al., Arch. Int. Pharmacodyn. Ther., 270 128, 1984).

Ethanol-Induced Ulcer Assay

Male Charles River CD rats weighing 250-280 g are fasted and deprived of water for 20 hours before the experiments. The test compound, homogenized in aqueous methyl cellulose vehicle, is administered orally 30 minutes prior to oral administration of 1 ml of absolute ethanol. One hour after ethanol, the rats are sacrificed and the stomachs excised. The stomachs are opened through the greater curvature and the length of each linear hemorrhagic lesion induced by ethanol is measured and summated for each stomach. Results are expressed as the mean lesion length (mm) per rat (± SE). The data are analyzed by Duncan's multiple range test and a P value of <.05 is considered significant.

The compounds of this invention are found to be effective at doses of about 0.05 - 50 mg/kg of body weight per day. Preferably the total dosages are administered in 2-4 divided doses per day.

When administered parenterally, e.g. intravenously, the compounds are administered at a dosage range of about 0.01 - 10 mg/kg of body weight in single or multiple daily doses.

To treat peptic ulcer disease, and prevent and treat drug-induced gastric ulceration, the active compounds of this invention can be administered in unit dosage forms such as tablets, capsules, pills, powders, granules, sterile parenteral solutions or suspensions, suppositories, mechanical delivery devices, e.g. transdermal, and the like.

The compounds of formula I are useful in the treatment of hyperproliferative skin-disease, e.g., psoriasis, which utility may be demonstrated by the Arachidonic Acid Mouse Ear Test described below.

Arachidonic Acid

Mouse Ear Test,

Materials and Methods

Charles River, female, CD, (SD) BR mice, 6 weeks old, are caged 8/group and allowed to acclimate 1-3 weeks prior to use.

Arachidonic acid (AA) is dissolved in reagent grade acetone (2 mg/.01 ml) and stored at -20 °C for a

maximum of 1 week prior to use. Inflammatory reactions are induced by applying 10 µl of AA to both surfaces of one ear (4 mg total).

Test drugs are dissolved in either reagent grade acetone or aqueous ethanol (only if insoluble in acetone) at the same doses selected by Opas et al., Fed. Proc. 43, Abstract 2983, p. 1927 (1984) and Young et al., J. Invest. Dermatol. 82, pp 367-371 (1984). These doses are employed to ensure maximum responses and to overcome any difference in topical absorption which could occur with any drug applied in an aqueous ethanol vehicle. The test drug is applied 30 minutes prior to challenge with AA.

The severity of the inflammation is measured as a function of increased ear weight. A 6 mm punch biopsy is removed 1 hour after AA challenge and weighed to the nearest 0.1 mg. Mean ± standard error and all possible comparisons are made via Duncan's Multiple Range Statistic. For instance, 1-methyl-1-(1,2-dihydro-4-hydroxy-1-phenyl-2-oxo-1,8-naphthyridin-3-yl)-pyrrolidinium-hydroxide, inner salt, when tested in the assay at a single dose of 0.5mg/ear, resulted in a 61% reduction of the severity of inflammation. At a dose of 0.1 mg/ear, the same compound gave a 43% reduction

When administered for the treatment of hyperproliferative skin disease, the compounds of formula I may be administered topically, orally, rectally or parenterally. When administered topically, the amount of compound administered varies widely with the amount of skin being treated, as well as with the concentration of active ingredient applied to the affected area. When administered orally, the compounds of formula I are effective for the treatment of hyperproliferative skin disease at daily doses ranging from about 0.1 mg/kg to about 100 mg/kg, preferably from about 5 mg/kg to about 50 mg/kg, which may be administered in divided doses. When administered rectally, the compounds of formula I may be administered in daily doses ranging from about 0.1 mg/kg to about 100 mg/kg. When administered parenterally, the compounds of formula I are effective for the treatment of hyperproliferative skin disease in daily doses ranging from about 0.1 mg/kg body weight to about 10 mg/kg body weight which may be administered in divided doses.

As a result of the topical administration of a compound of formula I, a remission of the symptoms of the psoriatic patient can in most cases be expected. Thus, one affected by psoriasis can expect a decrease in scaling, erythema, size of the plaques, pruritus and other symptoms associated with psoriasis. The dosage of medicament and the length of time required for successfully treating each individual psoriatic patient may vary, but those skilled in the art of medicine will be able to recognize these variations and adjust the course of therapy accordingly.

Included within the invention are preparations for topical application to the skin whereby the compounds having structural formula I are effective in the treatment and control of skin diseases characterized by rapid rates of cell proliferation and/or abnormal cell proliferation, e.g. psoriasis.

In a preferred method of carrying out the invention, a pharmaceutical formulation comprising a compound of formula I together with a non-toxic, pharmaceutically acceptable topical carrier, usually in concentrations in the range of from about 0.001 percent to about 10 percent, preferably from about 0.1 percent to about 5 percent, is applied several times daily to the affected skin until the condition has improved. Topical applications may then be continued at less frequent intervals (e.g. once a day) to control mitosis in order to prevent return of severe disease conditions.

For preparing pharmaceutical compositions from the compounds described by this invention, inert, pharmaceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

Also included are solid form preparations which are intended for conversion shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorings, colorants, stabilizers, buffers, artificial and natural sweeteners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

The compounds of the invention may also be deliverable transdermally. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation; for example, packaged tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and to the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

International Application No. PCT/US86/01518 (published as WO 87/00752) describes, as novel compounds, zwitterionic compounds falling within formula I (those wherein E is $-\overset{+}{N}R^1R^2R^3$ and $ZR^6$ is $Z^-$ ), but does not disclose their activity against hyperproliferative skin disease (e.g. psoriasis).

The following examples are intended to illustrate, but not to limit, the present invention.

## PREPARATIVE EXAMPLE 1

### Preparation of 4-hydroxy-1-phenyl-1,8-naphthyridin-2(1H)-one

A mixture of methyl-2-phenylamino nicotinate (75.2 g), n-butylacetate (700 mL) and potassium tert-butoxide (148 g) was stirred and heated gradually to reflux. The mixture was refluxed for 16 hours, after which time it was cooled and poured into water (7 L) with stirring. The resulting mixture was acidified to pH 5 with concentrated HCl when a white solid precipitated. The product was filtered off and air dried. The solid product was then suspended in hexane (3 L), triturated, filtered and washed with fresh hexane. This purification process was repeated using ether (1.5 L). The product was dried to yield 48 g of the desired

product, m.p. 312-314° C.

By a similar procedure, using modifications well known to one skilled in the art, the starting materials
ethyl-2-(pyrazinylamino)-nicotinate,
ethyl-2-(4-pyrimidinylamino)-nicotinate,
ethyl-2-(3-(1,2,4-triazinylamino))-nicotinate, and
ethyl-2-(2-thienylmethylamino)-nicotinate can to converted into
4-hydroxy-1-(2-pyrazinyl)-1,8-naphthyridin-2(1H)-one,
4-hydroxy-1-(4-pyrimidinyl)-1,8-naphthyridin-2(1H)-one,
4-hydroxy-1-(3-(1,2,4-triazinyl))-1,8-naphthyridin-2(1H)-one, and 4-hydroxy-1-(2-thienylmethyl)-1,8-naphthyridin-2(1H)-one, respectively.

## PREPARATIVE EXAMPLE 2

### Preparation of 3-bromo-4-hydroxy-1-phenyl-1,8-naphthyridin-2(1H)-one

To a suspension of 4-hydroxy-1-phenyl-1,8-naphthyridin-2(1H)-one (1 g) in $CH_2Cl_2$ (20 mL) was added, dropwise and with stirring, a solution of bromine (0.7 g) in $CH_2Cl_2$ (5 mL). The mixture was stirred at room temperature overnight, after which time the product was filtered off, dried in air and recrystallized from acetonitrile to yield 0.87 g of the product, m.p. 280° C.

By employing a similar procedure to that described in Preparative Example 2 above using simple modifications based on practices well-known to one skilled in the art, the compounds
4-hydroxy-1-(2-pyrazinyl)-1,8-naphthyridin-2(1H)-one,
4-hydroxy-1-(4-pyrimidinyl)-1,8-naphthyridin-2(1H)-one,
4-hydroxy-1-(3-(1,2,4-triazinyl))-1,8-naphthyridin-2(1H)-one, and
4-hydroxy-1-(2-thienylmethyl)-1,8-naphthyridin-2(1H)-one
can be converted into
3-bromo-4-hydroxy-1-(2-pyrazinyl)-1,8-naphthyridin-2(1H)-one,
3-bromo-4-hydroxy-1-(4-pyrimidinyl)-1,8-naphthyridin-2(1H)-one,
3-bromo-4-hydroxy-1-(3-(1,2,4-triazinyl))-1,8-naphthyridin-2(1H)-one, and
3-bromo-4-hydroxy-1-(2-thienylmethyl)-1,8-naphthyridin-2(1H)-one, respectively.

## PREPARATIVE EXAMPLE 3

### Preparation of 1-(1,2-dihydro-4-hydroxy-1-phenyl-2-oxo-1,8-naphthyridin-3-yl)-1-methyl-pyrrolidinium hydroxide, inner salt

In dry pyridine (30 mL), 3-bromo-4-hydroxy-1-phenyl-1,8-naphthyridin-2(1H)-one (10 g) was suspended. N-methyl pyrrolidine (20 mL) was added to the suspension. The mixture was heated to 95-100° C with stirring, and was kept there for about 33 hours. The product was evaporated under high vacuum to provide a dark oil. This oil was slurried with 200 mL of $CH_3CN$(40): $H_2O$(60): $CH_3CO_2H$(1) and filtered. The solid residue on the filter was rinsed with water and the filtrate was evaporated to remove most of the $CH_3CN$. Reversed phase chromatography through an E. Merck RP-8 LoBar column, eluting with increasing concentrations of $CH_3CN$ in $H_2O$ (containing 1% $CH_3CO_2H$) gave a moderately pure product which was subjected to a second chromatographic separation using the same conditions as above. Fractions containing the product were combined and evaporated to yield a solid which was recrystallized from $CH_2Cl_2$/isopropanol to yield the desired product, m.p. 245-250° C.

## PREPARATIVE EXAMPLE 4

Preparation of 1-(1,2-dihydro-4-hydroxy-1-phenyl-2-oxo-1,8-naphthyridin-3-yl)-1-methyl-pyrrolidinium chloride

1-(1,2-dihydro-4-hydroxy-1-phenyl-2-oxo-1,8-naphthyridin-3-yl)-1-methyl-pyrrolidinium hydroxide, inner salt (0.1 g) was dissolved in 0.1 N-HCl solution (38 mL). The solution was concentrated under high vacuum to provide an oil which crystallized on the addition of isopropanol. The solid was filtered off and washed with isopropanol to yield the desired hydrochloride salt, m.p. 195 °C.

## PREPARATIVE EXAMPLE 5

Preparation of 1-(1,2-dihydro-4-hydroxy-1-phenyl-2-oxo-1,8-naphthyridin-3-yl)-pyrrolidinium hydroxide, inner salt

A solution of 3-bromo-4-hydroxy-1-phenyl-1,8-naphthyridin-2(1H)-one (2 g) in a mixture of pyrrolidine (10 mL) and DMF (5 mL) was stirred and heated at 100 °C for 2 days. The resulting mixture was then cooled, diluted with $CH_2Cl_2$ (100 mL) and filtered. The solid was triturated with hot $CHCl_3$, filtered, and dried to yield the desired product, m.p. 282-284 °C.

Some of the product was purified by (1) dissolving in a minimum volume of 2,2,2-trifluoroethanol and (2) precipitating by addition of 4 volumes of methanol. The pure product charred and decomposed when heated above about 285 °C.

## PREPARATIVE EXAMPLE 6

Preparation of 1-(1,2-dihydro-4-hydroxy-1-phenyl-2-oxo-1,8-naphthyridin-3-yl)-4-hydroxy-piperidinium hydroxide, inner salt

A solution of 3-bromo-4-hydroxy-1-phenyl-1,8-naphthyridin-2(1H)-one (1 g) in a mixture of 2,6-lutidine (5 mL) and 4-hydroxy-piperidine (3.12 g) was heated at 100 °C for 32 hours. The lutidine was removed by evaporation under high vacuum. The residue was dissolved in $CH_3CN$(20): $H_2O$(80): $CH_3CO_2H$(1) and separated by reversed phase preparative HPLC (Whatman Magnum 40 with Partisil 40/ODS-3). The fractions containing the desired product were combined and evaporated to yield a partially crystalline material which was recrystallized from isopropanol to yield the desired product, m.p. 256-258 °C.

The following compounds were also prepared by the techniques similar to those described above:

1-(1,2-dihydro-4-hydroxy-1-phenyl-2-oxo-1,8-naphthyridin-3-yl)quinuclidinium hydroxide, inner salt, hemihydrate, m.p. 290 °C.

1-methyl-1-(1,2-dihydro-4-hydroxy-1-phenyl-2-oxo-1,8-naphthyridin-3-yl)-morpholinium hydroxide, inner salt, hemihydrate, m.p. 248-249 °C.

1-(1,2-dihydro-4-hydroxy-1-phenyl-2-oxo-1,8-naphthyridin-3-yl)-piperidinium hydroxide, inner salt, hemihydrate, m.p. 261-263 °C (decomp.).

1-(1,2-dihydro-4-hydroxy-1-phenyl-2-oxo-1,8-naphthyridin-3-yl)-2-hydroxymethyl-piperidinium hydroxide, inner salt, hemihydrate, m.p. 135-138 °C.

1-(5,6-dihydro-8-hydroxy-6-oxo-5-phenyl-pyrido[2,3-b]pyrazin-7-yl)-pyrrolidinium-hydroxide, inner salt, 1/4 hydrate, m.p. >260 °C.

4-acetyl-1-(1,2-dihydro-4-hydroxy-2-oxo-1-phenyl-1,8-naphthyridin-3-yl)-piperazinium hydroxide, inner salt, 2-propanolate, m.p. 244-246.5 °C.

1-(5,6-dihydro-8-hydroxy-6-oxo-5-phenyl-pyrido[2,3-b]pyrazin-7-yl)-4-(2-hydroxy-ethyl)-piperazinium hydroxide, inner salt, m.p. 258-260 °C.

By employing procedures similar to those described above in Preparative Examples 3, 4, 5 and 6 with simple modifications well known to one skilled in the art, the compounds 3-bromo-4-hydroxy-1-(2-pyrazinyl)-1,8-naphthyridin-2(1H)-one,

3-bromo-4-hydroxy-1-(4-pyrimidinyl)-1,8-naphthyridin-2(1H)-one,
3-bromo-1-(3-chlorophenyl)-4-hydroxy-1,8-naphthyridin-2(1H)-one,
3-bromo-4-hydroxy-1-(3-(1,2,4-triazinyl))-1,8-naphthyridin-2(1H)-one, and
3-bromo-4-hydroxy-1-(2-thienylmethyl)-1,8-naphthyridin-2(1H)-one
can be converted into
1-[1,2-dihydro-4-hydroxy-2-oxo-1-(2-pyrazinyl)-1,8-naphthyridin-3-yl]-1-methyl-pyrrolidinium hydroxide, inner salt,
1-[1,2-dihydro-4-hydroxy-2-oxo-1-(4-pyrimidinyl)-1,8-naphthyridin-3-yl]-1-methyl-piperidinium hydroxide, inner salt,
1-[1-(3-chlorophenyl)-1,2-dihydro-4-hydroxy-2-oxo-1,8-naphthyridin-3-yl]-piperidinium hydroxide, inner salt, m.p. 258.5-261 °C,
1-[1,2-dihydro-4-hydroxy-2-oxo-1-(3-(1,2,4-triazinyl))-1,8-naphthyridin-3-yl]-pyrrolidinium hydroxide, inner salt, and
1-(1,2-dihydro-4-hydroxy-2-oxo-1-(2-thienylmethyl)-1,8-naphthyridin-3-yl]-piperidinium hydroxide, inner salt, respectively.

## PREPARATIVE EXAMPLE 7

Preparation of 4-acetyloxy-1-phenyl-3-(1-pyrrolidinyl)-1,8-naphthyridin-2(1H)-one

A mixture of 1-(1,2-dihydro-4-hydroxy-1-phenyl-2-oxo-1,8-naphthyridin-3-yl)-pyrrolidinium hydroxide, inner salt (3g) and triethylamine (1.41g) in $CH_2Cl_2$ (50 mL) was stirred at room temperature in an atmosphere of nitrogen. Acetyl chloride (1.09g) was added dropwise and stirring was continued for a total of about 20-24 hours. The product was poured into water (200 mL) and the pH was adjusted to 7 with 10% KOH solution. The organic layer was separated and dried ($Na_2SO_4$). The dry solution was filtered and evaporated to a solid which was recrystallized from isopropanol to yield the desired product, 2.17g (67%) m.p. 197-200 °C.

## PREPARATIVE EXAMPLE 8

Preparation of 4-(2,2-dimethylpropionyloxy)-1-phenyl-3-(1-pyrrolidinyl)-1,8-naphthyridin-2(1H)-one

A mixture of 1-(1,2-dihydro-4-hydroxy-1-phenyl-2-oxo-1,8-naphthyridin-3-yl)-pyrrolidinium hydroxide, inner salt (3g) and triethylamine (1.88g) in $CH_2Cl_2$ was stirred under $N_2$ for about 1 hour at room temperature. Trimethylacetyl chloride (1.4g) was added and stirring was continued for about a total of 20 hours.

The product was poured into ice/water and the pH was adjusted to 5 with acetic acid. The aqueous phase was extracted with ethyl acetate, the combined organic layers were dried ($MgSO_4$), filtered and evaporated to a solid which was crystallized from isopropanol to yield the desired product, 2.75g (66%) m.p. 163-165 °C.

## EXAMPLE 1

Preparation of 4-(4-methylbenzoyloxy)-1-phenyl-3-(1-pyrrolidinyl)-1,8-naphthyridin-2(1H)-one

A mixture of 1-(1,2-dihydro-4-hydroxy-1-phenyl-2-oxo-1,8-naphthyridin-3-yl)-pyrrolidinium hydroxide, inner salt (3.0g) and triethylamine (1.88g) in $CH_2Cl_2$ (100 mL) was stirred for 1 hour at room temperature. p-Toluoyl chloride (1.79g) was added and the mixture was stirred at room temperature for about 24 hours.

The product was poured into water (200 L) and the pH was adjusted to about 6 with acetic acid. The organic layer was separated, the aqueous layer was extracted with ethyl acetate, and the organic layers were combined, dried ($Na_2SO_4$), filtered and evaporated to a solid which was recrystallized from isopropanol to yield the desired product, 2.07g (50%), m.p. 216-217° C.

## EXAMPLE 2

Preparation of 4-(N,N-diethylcarbamoyloxy)-1-phenyl-3-(1-pyrrolidinyl)-1,8-naphthyridin-2(1H)-one

A mixture of 1-(1,2-dihydro-4-hydroxy-1-phenyl-2-oxo-1,8-naphthyridin-3-yl)-pyrrolidinium hydroxide, inner salt (3g), triethylamine (1.88g), and diethylcarbamoyl chloride (1.57g) in dry pyridine (50 mL) was stirred overnight at room temperature in an $N_2$ atmosphere. Some starting material remained so dimethylformamide (50 mL), 1,8-diazabicyclo[5.4.0]undec-7-ene (DBU) (2.83g), and diethylcarbamoyl chloride (1.57g) were added and the mixture was heated at 80° C for 0.5 hour. The reaction mixture was then poured over ice and the pH was adjusted to about 6 with acetic acid. The resulting solution was extracted three times with ethyl acetate. The organic extracts were combined, dried ($Na_2SO_4$), filtered and evaporated to a solid which was recrystallized from isopropanol to yield the desired product, 2.03g (54%), m.p. 158-160° C.

## EXAMPLE 3

Preparation of 4-(N,N-dimethylcarbamoyloxy)-1-phenyl-3-(1-pyrrolidinyl)-1,8-naphthyridin-2(1H)-one

To a suspension of 1-(1,2-dihydro-4-hydroxy-1-phenyl-2-oxo-1,8-naphthyridin-3-yl)-pyrrolidinium hydroxide, inner salt (5.0g) in dry pyridine (80 mL) and an $N_2$ atmosphere, was added DBU (4.71g) and dimethylcarbamoyl chloride (2.08g). The mixture was heated to 80° C for 0.5 hour after which time it was poured into ice/water. The pH was adjusted to 6 with acetic acid. The organic layer was separated and the aqueous layer was extracted three times with ethyl acetate (200 mL). The combined organic layers were dried ($Na_2SO_4$), filtered and evaporated to a solid. This material was suspended in ether (1L) and to it was added, with stirring, 1.5 equivalent of methanesulfonic acid in ether (500 mL). After 1 hour of vigorous stirring the solid product was filtered off, washed with ether and recrystallized from isopropanol to yield the salt of the desired product, 5.05g.

This material was suspended in water and aqueous $NaHCO_3$ solution was added until the pH of the water was about 6. The product was filtered off and dried to provide the desired product in 46% yield, m.p. 218-220° C.

## EXAMPLE 4

PREPARATION OF 4-HYDROXY-1-PHENYL-3-(1-PYRROLIDINYL)-1,8-NAPHTHYRIDIN-2-(1H)-ONE

To a suspension of 1.4 g (4.1 millimoles (mM)) of 3-pyridinecarboxylic acid 2-{[(1-pyrrolidinyl)acetyl] phenylamino }methyl ester in t-butylmethylether at 0° -5° C, 1.03 g (9.2 mM) potassium-t-butoxide is added. The reaction mixture is stirred for an additional 0.5 hour at 0-5° C and allowed to warm up to room temperature. Next 0.75 mL glacial acetic acid is added very slowly. The resultant solid is filtered off, washed with t-butylmethylether, methylene chloride, acetone, water and acetone. The product is air-dried to give 0.94 g (73% yield) of title compound, a white solid.

EXAMPLE 5

PREPARATION OF 4-HYDROXY-1-PHENYL-3-(1-PYRROLIDINYL)-1,8-NAPHTHYRIDIN-2-(1H)-ONE

To a solution of 1.5 g (6.5 mM) methyl 2-phenylaminonicotinate in dry xylenes at room temperature is added 0.69 g (14.54 M) of sodium hydride (50 percent oil emulsion) followed by a small amount of N,N-dimethylformamide (DMF). The reaction mixture is heated to a temperature of 85-95°C, and 1.05 mL (6.5 mM) of ethyl 1-pyrrolidineacetate in xylene is slowly added over a period of 10 minutes. The reaction mixture is heated for 1 to 3 hours prior to the addition of portions of 0.32 g NaH followed by 1.05 mL of ethyl 1-pyrrolidineacetate as described above (total 3 portions). Following addition of the portions, the reaction mixture is cooled to 0°C and quenched with a slow addition of glacial acetic acid, and then water is added. The product is filtered off and washed with water, acetone, methylene chloride, and acetone. The so-obtained solid is dried in vacuo to give 1.20 g (60% yield) of title compound, a white solid.

EXAMPLE 6

PREPARATION OF METHYL 2-[(CHLOROACETYL)PHENYLAMINO]-PYRIDINE-3-CARBOXYLATE

To a stirred solution of 26.3 g methyl 2-phenylaminonicotinate (11.5 mM) in t-butylmethylether at 50°C (oil bath) under nitrogen atmosphere, 20.2 mL chloroacetylchloride (25.39 mM) is added, followed by 32.4 mL propylene oxide (46 mM). The reaction mixture is stirred at 50°C for 2 additional hours, cooled to room temperature, diluted with t-butylmethyl ether and washed with water containing $NaHCO_3$. The layers are separated, the aqueous layer is extracted with t-butylmethylether, the combined organic layers are dried over anhydrous $Na_2SO_4$ and concentrated in vacuo to obtain a gummy solid which is recrystallized from t-butylmethyl ether to give 30.5 g (87% yield) of title compound, an off-white solid.

IR ($CHCl_3$) 1700,1740 $cm^{-1}$,

NMR ($CDCl_3$) w 4.1 (chloromethyl).

EXAMPLE 7

PREPARATION OF 1-(1,2-DIHYDRO-4-HYDROXY-1-PHENYL-2-OXO-1,8-NAPHTHYRIDIN-3-YL)-PYRROLIDINIUM HYDROXIDE, INNER SALT

Step A: To a stirred solution of 25.45 g (0.11M) of methyl 2-phenylamino-nicotinate in 160 mL of t-butylmethylether (tBuOMe) (dried over 3A° sieves) heated to 50° (under $N_2$), 19.5 mL (2.2 x 0.11M) of chloroacetylchloride was added, followed by 31 mL (4 x 0.11m) of propylene oxide. The reaction mixture was heated at 50°C for 1.5 hours and then 300 mL tBuOMe was added. This solution (cooled to room temperature) was washed with 200 mL $H_2O$ containing 9.37 g (0.11M) of $NaHCO_3$ followed by 30 mL of saturated aqueous NaCl solution. At this stage the product that crystallized out was dissolved in 100 mL $CH_2Cl_2$ and this $CH_2Cl_2$ was mixed with tBuOMe. The solution was used as such in the next step.

Step B: To the above solution at room temperature under $N_2$, 37.2 mL (4 x 0.11M) of pyrrolidine was added and this solution was gently refluxed overnight. 9.3 mL (0.11M) of pyrrolidine was added, and the reaction was refluxed for an additional two hours. This mixture was diluted with 600 mL tBuOMe and washed with 300 mL $H_2O$, and the aqueous layers were back-extracted with 200 mL tBuOMe. The combined organic (tBuOMe) layer was washed with 150 mL saturated aqueous NaCl solution, dried over anhydrous $Na_2SO_4$, and then concentrated in vacuo (oil pump vacuum) to yield 64.6 g of a brown semisolid, crude methyl 2-[(1-pyrrolidinylacetyl)phenylamino]-pyridine-3-carboxylate.

Step C: The solid from step B above was suspended in 600 mL of cold (0 ° C) tBuOMe (dried over 3A ° sieves) under $N_2$. To this cold stirred mixture, 27.5 g (2.2 x 0.11M) potassium t-butoxide was added, the reaction mixture was stirred for 1 hour, and then it was quenched with 15 mL (2.4 x 0.11M) of glacial acetic acid.

The stirred reaction mixture was allowed to attain room temperature and then 350 mL $H_2O$ was added to it. The resultant solid was filtered off, washed with tBuOMe, $H_2O$, a small amount of $CH_2Cl_2$, and acetone, and then air-dried to yield 27.09 g of the white product 1-(1,2-dihydro-4-hydroxy-1-phenyl-2-oxo-1,8-naphthyridin-3-yl)-pyrrolidinium hydroxide, inner salt. The crude product was dissolved in 300 mL $CH_3OH$ + 16 mL conc. $H_2SO_4$ at 50 ° C with 3g carbon; the solution was filtered, diluted with 575 mL $H_2O$, cooled to 0 ° C and filtered; the product was dried in a draft oven at 60 ° C for about 18 hours to give 22.2 g (82%) of crystalline white product.

## PREPARATION OF STARTING MATERIALS

The starting materials employed in inventive processes A, B and C are known or can be prepared from known procedures. See, for example, United States patents 4,684,727, 4,452,800, 4,492,702 and 4,680,298, whose preparative teachings are incorporated herein by reference.

## EXAMPLE 8

## PREPARATION OF METHYL 2-{[(1-PYRROLIDINYL)ACETYL]PHENYLAMINO}-PYRIDINE-3-CARBOXYLATE

To a gently refluxing stirred solution of 1g (3.3 mM) of methyl 2-[(chloroacetyl)phenylamino]-pyridine-3-carboxylate in t-butylmethyl ether, 1.1 mL pyrrolidine (13.2 mM) is added. The reaction mixture is refluxed for 2.5 hours, diluted with t-butylmethyl ether, and washed with water. The water layer is extracted with t-butylmethyl ether, and the combined organic phases are washed with a saturated aqueous sodium chloride (NaCl) solution, dried over anhydrous sodium sulfate ($Na_2SO_4$) and then concentrated in vacuo to give 1.1 g (93% yield) of title compound, a tan solid.

IR ($CHCl_3$) 1685, 1725 $cm^{-1}$,

NMR ($CDCl_3$) $\delta$ 3.25 (N-CO-$CH_2$-N).

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. The use of a compound of the formula I

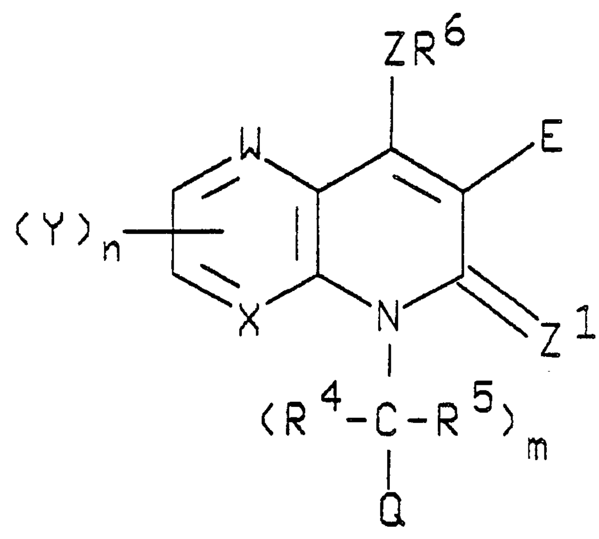

wherein:

W and X may be the same or different and each independently represents -CH= or -N=;

$Z^1$ and Z are the same or different and each independently represents O or S;

E is a group $-NR^1R^2$ or $-\overset{+}{N}R^1R^2R^3$;

$R^1$, $R^2$, $R^3$, $R^4$ and $R^5$ are the same or different and each may be independently selected from H, alkyl having from 1 to 12 carbon atoms, alkenyl having from 3 to 8 carbon atoms, alkynyl having from 3 to 8 carbon atoms, alkoxyalkyl having from 1 to 6 carbon atoms in the alkoxy portion and from 2 to 6 atoms in the alkyl portion thereof, hydroxyalkyl having from 2 to 8 carbon atoms, cycloalkyl having from 3 to 8 carbon atoms, acyloxyalkyl having from 1 to 6 carbon atoms in the acyloxy portion and from 2 to 8 carbon atoms in the alkyl portion thereof, and $-R^7-CO_2R^0$ wherein $R^7$ represents an alkylene group having from 1 to 6 carbon atoms and $R^0$ represents hydrogen or an alkyl group having from 1 to 6 carbon atoms, with the provisos that the OH of the hydroxyalkyl group and the acyloxy of the acyloxyalkyl group are not joined to the same carbon atom as another heteroatom and that, when $R^1$, $R^2$ and/or $R^3$ are alkenyl or alkynyl, there is at least one carbon-carbon single bond between the nitrogen atom and the carbon-carbon double or triple bond;

in addition, one of $R^2$ and $R^3$ can be an aryl group or an aromatic heterocyclic group, either of which can be substituted with one to three substituents Y as defined below;

in further addition, any two of $R^1$, $R^2$ and $R^3$, together with the adjacent nitrogen atom, can be joined together to represent a ring which can contain from 2 to 8 carbon atoms, said ring optionally containing an -O-, -S- and/or $-NR^4-$ heteroatomic group (wherein $R^4$ is as defined above) and/or optionally containing a carbon-carbon double bond, said ring optionally being substituted with one to three additional substituents $R^8$ which may be the same or different and are each independently selected from OH with the proviso that OH is not on a carbon already joined to a hetero atom, -O-acyl having from 1 to 6 carbon atoms, hydroxyalkyl having from 1 to 8 carbon atoms, alkoxyalkyl having from 1 to 6 carbon atoms in the alkyl and alkoxy portions thereof, alkyl having from 1 to 6 carbon atoms, alkenyl having from 3 to 8 carbon atoms, alkynyl having from 3 to 8 carbon atoms, and $-COOR^9$ wherein $R^9$ represents H, alkyl or aryl, or any two $R^8$ substituent groups may combine to form a hydrocarbon ring having from 4 to 8 total carbon atoms;

in still further addition, all three of $R^1$, $R^2$ and $R^3$ can be joined together to represent a polycyclic hydrocarbon ring, which polycyclic ring can optionally be substituted by one to three substituent groups $R^8$ as defined above;

$R^6$ represents alkanoyl, $-CO-R^{10}$, $-CS-OR^{17}$, $-CS-NR^{15}R^{16}$, $-C(R^{11})_2-OR^{12}$, $-C(R^{11})_2-SR^{12}$ or $-C(R^{11})_2-NR^{12}R^{13}$ or, when E is $-\overset{+}{N}R^1R^2R^3$, $R^6$ represents $A^-$ or any of the foregoing groups together with $A^-$;

$R^{10}$ represents aryl, $-R^{14}$, aromatic heterocyclic, $-OR^{14}$ or $-NR^{15}R^{16}$;

each $R^{11}$ represents H, alkyl, $-CCl_3$, $-COOR^9$ or aryl;

$R^{12}$ represents $-R^{14}$, $-CO-R^{13}$ or $-CS-R^{17}$;

$R^{13}$ represents H, alkyl or aryl;

$R^{14}$ represents alkyl of from 1 to 12 carbon atoms;

$R^{15}$ and $R^{16}$ each independently represents H, alkyl or aryl, or $R^{15}$ and $R^{16}$ together represent a divalent polymethylene group of from 4 to 6 carbon atoms, said polymethylene group being optionally substituted with a carboxy group or alkyl ester thereof;

$R^{17}$ represents $-R^{14}$ or aryl;

m is an integer of from 0 to 3;

n is an integer of from 0 to 2;

Q represents an aryl or an aromatic heterocyclic group which can optionally be substituted with 1 to 3 substituents Y as defined below;

each Y substituent is independently selected from hydroxy, alkyl having from 1 to 6 carbon atoms, halogen, $NO_2$, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, hydroxyalkyl having from 1 to 6 carbon atoms, $-S(O)_n-R^{18}$ (wherein $R^{18}$ represents alkyl having from 1 to 6 carbon atoms and n is as defined above), $-SO_2NH_2$, $-CO-R^{19}$ (wherein $R^{19}$ represents OH, $-NH-R^{18}$ or $-O-R^{18}$, where $R^{18}$ is as defined above), $-O-B-(O)_p-COR^{19}$ (wherein B represents an alkylene group having from 1 to 4 carbon atoms, p is 0 or 1, and $R^{19}$ is as defined above), $-NH_2$, -NHCHO, $-NH-CO-R^{29}$ (wherein $R^{19}$ is as defined above, with the proviso that it is not hydroxy), $-NH-COCF_3$, $-NH-SO_2R^{18}$ (wherein $R^{18}$ is as defined above), and $-NHSO_2CF_3$;

$A^-$ is one equivalent of a pharmaceutically acceptable anion or, when $R^6$ represents $A^-$ alone, then $A^-$ is an electron on Z;

or of a pharmaceutically acceptable salt theeof with a base when $ZR^6$ represents $Z^1$ ;

for the preparation of pharmaceutical compositions useful in the treatment of hyperproliferative skin disease.

2. A compound having the structural formula Ia or Ib

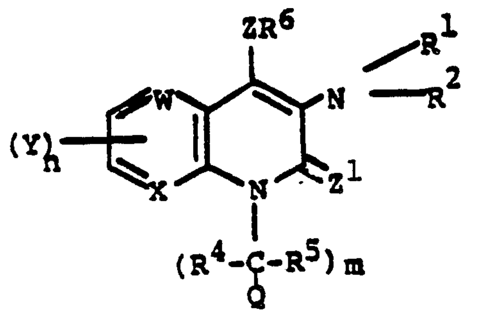

or

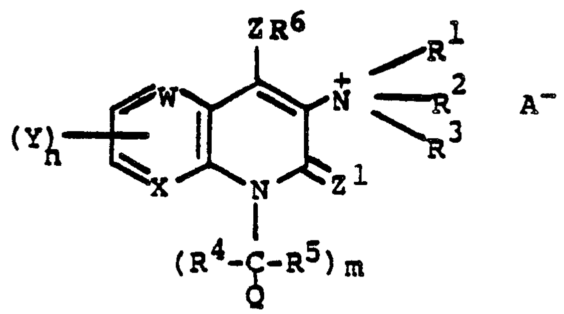

(Ia) (Ib)

wherein:

$R^6$ represents $-CO-R^{10}$, $-CS-OR^{17}$, $-CS-NR^{15}R^{16}$, $-C(R^{11})_2-OR^{12}$, $-C(R^{11})_2-SR^{12}$ or $-C(R^{11})_2-NR^{12}R^{13}$;

and W, X, Y, $Z^1$, Z, $R^1$, $R^2$, $R^3$, $R^4$, $R^5$, $R^{10}$, $R^{11}$, $R^{12}$, $R^{13}$, $R^{15}$, $R^{16}$ ,$R^{17}$, m, n, Q and $A^-$ are as defined in claim 1.

3. A compound according to claim 2, wherein at least one of W and X is -N=, and preferably wherein X is -N= and W is -CH=.

4. A compound according to claim 2 or claim 3, wherein at least one of $Z^1$ and Z is 0, and preferably both $Z^1$ and Z are 0.

5. A compound according to any of claims 2 to 4, wherein at least one of n and m is zero, and preferably both are zero.

6. A compound according to any of claims 2 to 5, wherein Q is a phenyl group which may optionally be substituted with one or two Y groups.

7. A compound according to claim 2, selected from:

4-(4-methylbenzoyloxy)-1-phenyl-3-(1-pyrrolidinyl)-1,8-naphthyridin-2(1H)-one;

4-(N,N-diethylcarbamoyloxy)-1-phenyl-3-(1-pyrrolidinyl)-1,8-naphthyridin-2(1H)-one;

4-(N,N-dimethylcarbamoyloxy)-1-phenyl-3-(1-pyrrolidinyl)-1,8-naphthyridin-2(1H)-one;

and the pharmaceutically acceptable salts and solvates of said compounds.

8. A pharmaceutical composition which comprises a compound having structural formula Ia or Ib as defined in claim 2 in combination with a pharmaceutically acceptable carrier.

9. A method for treating asthma/allergic reactions in a mammal, or a method for treating inflammation in a mammal, or a method for treating peptic ulcers in a mammal, which comprises administering a compound of formula Ia or Ib as defined in claim 2 or a pharmaceutical composition containing it to said mammal, in an amount sufficient to provide respectively an anti-asthma/anti-allergic effect, an anti-inflammatory effect, or a cytoprotective effect.

10. A method for treating a mammal suffering from hyperproliferative skin disease which comprises administering an effective amount of a compound having structural formula I as defined in claim 1, or of a pharmaceutically acceptable salt thereof with a base when $ZR^6$ represents $Z^-$, to said mammal.

11. A process for the preparation of a bicyclic compound of the formula

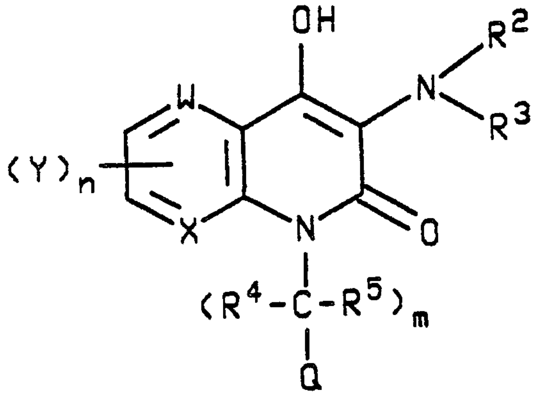

(X)

wherein W, X, $R^2$, $R^3$, $R^4$, $R^5$, m, n, Q and Y are as defined in claim 1;

which comprises the step of cyclising, in the presence of a base and a solvent, a compound of the formula

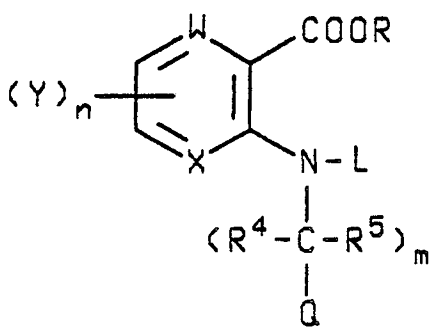

(VA)

wherein L is H or a group of the formula

$-CO-CH_2-NR^2R^3$ (VB),

$R^2$, $R^3$, $R^4$, $R^5$, Q, W, X, Y, m and n are as defined in claim 1,

and R is any of the values for $R^4$ or $R^5$ with the proviso that R is not hydrogen,

intramolecularly when L represents the group of the formula (VB),

and intermolecularly with an amino-substituted acetic acid derivative of the formula

$ROOC-CH_2-NR^2R^3$ (XI)

wherein R, $R^2$ and $R^3$ are as defined hereinbefore, when L represents H,

to yield a compound of formula (XII) defined above.

12. A process as claimed in claim 11 for preparing a compound of the formula

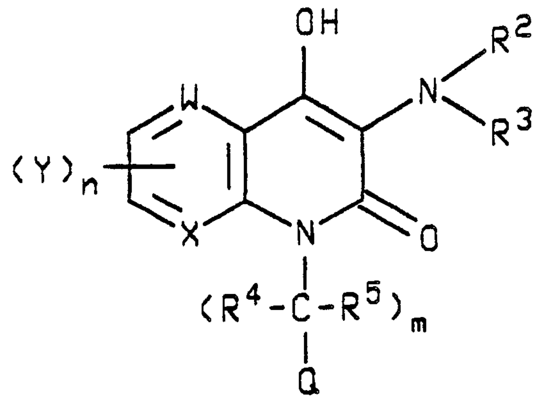

(X)

wherein W, X, $R^2$, $R^3$, $R^4$, $R^5$, m, n, Q and Y are as defined in claim 1,

with the proviso that $R^2$ and $R^3$ and the adjacent N atom can be joined together to represent a polycyclic ring, which can optionally be substituted by one to three substituents $R^7$ as defined in claim 1;

comprising contacting an amino acetamide compound of the formula:

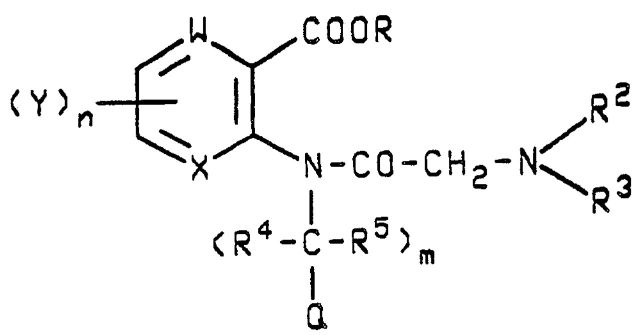

(VC) ,

wherein Y, W, X, $R^2$, $R^3$, $R^4$, $R^5$, Q, m and n are as defined in claim 1, and

R is any of the values for $R^4$ or $R^5$ with the proviso that R is not hydrogen,

with a base effective to remove a proton selectively from the methylene group of said amino acetamide compound (VC) in order to cyclize said compound intramolecularly to the bicyclic compound of formula (X).

13. A process as claimed in claim 11 for preparing a bicyclic compound of the formula

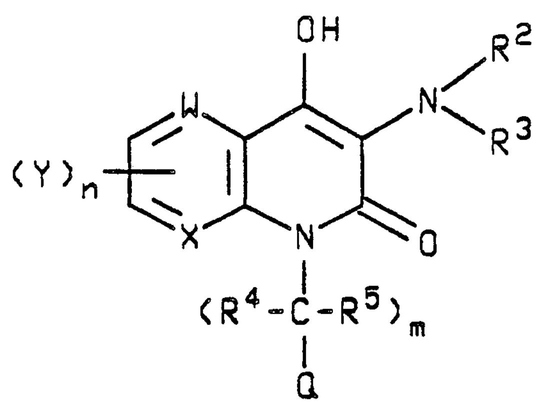

(X)

wherein W, X, $R^2$, $R^3$, $R^4$, $R^5$, m, n, Q and Y are as defined in claim 1,

with the proviso that $R^2$ and $R^3$ and the adjacent N atom can be joined together to represent a polycyclic ring, which can optionally be substituted by one to three substituents $R^8$ as defined in claim 1;

comprising contacting a secondary substituted amine of the formula:

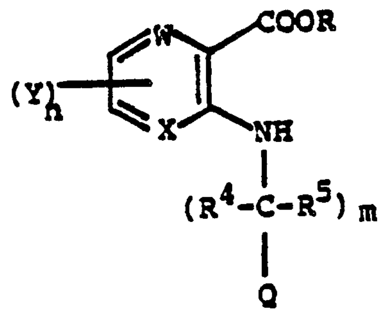

(V)

wherein Y, W, X, $R^2$, $R^4$, $R^5$, Q, m and n are as defined hereinbefore; and R independently represents the values for $R^4$ and $R^5$ with the proviso that R is not hydrogen,

with an amino-substituted acetic acid derivative of the formula:

$ROOC-CH_2-NR^2R^3$ (XI)

wherein $R^2$ and $R^3$ are as defined in claim 1; and R is as defined above;

with a base effective to cyclize said secondary substituted amine (V) with said amino-substituted acetic acid derivative (XI) to give the desired bicyclic compound (X).

14. A process for preparing a substituted acetamide compound of the formula

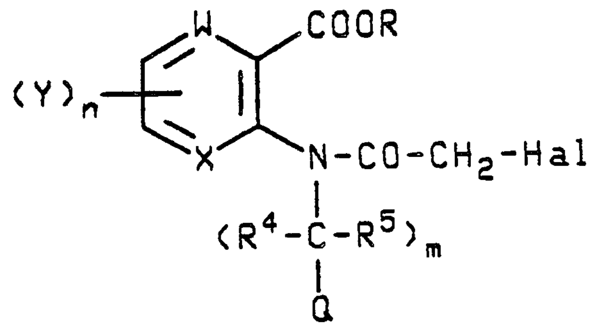

(XII)

wherein W, X, Y, $R^4$, $R^5$, m, n, and Q are as defined in claim 1, and R is as defined in claim 13;

which comprises contacting a secondary amine compound of the formula:

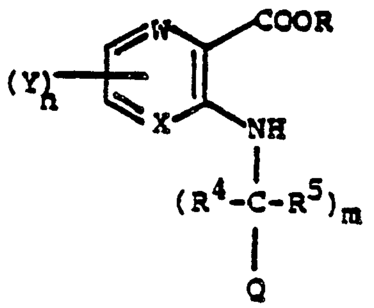

(V)

wherein Y, W, X, $R^4$, $R^5$, m and n are as defined in claim 1, and R is as defined in claim 13;
with a substituted acetic acid derivative of the formula

$Hal-CH_2COLg$

wherein Hal is halogen and Lg is a leaving group;
in the presence of a proton accepting compound.

15. A process for the preparation of esters, acetal ethers, thioethers and nitrogen-substituted derivatives of the formula I defined in claim 1, which comprises reacting a compound of structural formula VIII with a compound of formula IX:

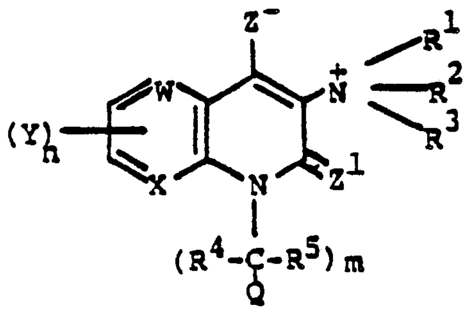

$R^6Lg$

(IX)

VIII

VIII wherein $R^1$, $R^2$, $R^3$, $R^4$, $R^5$, $R^6$, Q, X, Y, W, $Z^1$ , Z, m and n are as defined in claim 1, except that $R^6$ is not an alkanoyl group and neither represents nor includes $A^-$ , and Lg is an appropriate leaving group.